# EUROPEAN PATENT APPLICATION

(11) **EP 4 710 979 A2**
(43) Date of publication of application: **18.03.2026**
(21) Application number: 25211562.1
(22) Date of filing: 08.10.2021
(51) Int. Cl.: A61M 16/20

(54) **HEAT AND MOISTURE EXCHANGER WITH FLEXIBLE FRAME FOR PATIENT INTERFACE**

(30) Priority: 09.10.2020 AU 2020903663
(62) Divisional of application: 21876768.9
(71) Applicant: ResMed Pty Ltd, Bella Vista, NSW 2153 (AU)
(72) Inventor: SHORT, Skye Kimberly, Bella Vista, New South Wales 2153 (AU)
(74) Representative: Vossius & Partner Patentanwälte Rechtsanwälte mbB

(57) **Abstract**

The present invention relates to a vent and heat and moisture exchanger (HMX) assembly comprising: a vent assembly configured to be connected to a plenum chamber of a patient interface and having a plurality of vent holes configured to allow a continuous flow of gases exhaled by a patient from within the plenum chamber to ambient throughout the patient's respiratory cycle, the vent holes being sized and shaped to maintain a therapeutic pressure in the plenum chamber in use; and an HMX assembly connected to the vent assembly such that the HMX assembly and the vent assembly are removable from the plenum chamber together as a single unit, the HMX assembly including an HMX frame and a material positioned on the HMX frame, the material (3850) being configured to adsorb water vapour from gases exhaled by the patient and desorb water vapour into a flow of air at the therapeutic pressure, the material being supported by the HMX frame such that when the vent assembly is connected to the plenum chamber, the material is positioned within the plenum chamber to allow at least a portion of the flow of air at the therapeutic pressure to pass through the material before entering the patient's airways, wherein the HMX frame is constructed from a flexible material.

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

### 1 CROSS-REFERENCE TO RELATED APPLICATIONS

This application claims priority to Australian Provisional Application No. 2020903663, filed October 9, 2021, the entire contents of which are incorporated herein by reference.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nares and the mouth are the entrances to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology",* by John B. West, Lippincott Williams & Wilkins, 9th edition published 2012.

A range of respiratory disorders exist. Certain disorders may be characterised by particular events, e.g., apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. *See* Sullivan, U.S. Patent No. 4,944,310.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.3 Respiratory therapy systems

These respiratory therapies may be provided by a respiratory therapy system or device. Such systems and devices may also be used to screen, diagnose, or monitor a condition without treating it.

A respiratory therapy system may comprise at least one of: a Respiratory Pressure Therapy (RPT) device, an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may seal, e.g., with a region of the patient's face, to facilitate the delivery of gas at a pressure at sufficient variance with ambient pressure to effect therapy, e.g., at a positive pressure of about 10 cmH₂O relative to ambient pressure.

For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid complete sealing. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology, e.g., if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g., for sleeping while lying on one's side in bed with the head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable, especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g., filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable or difficult to use, a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g., aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to, for example, overlay an upper lip region and a nasal bridge region of a face. In one form of patient interface, a seal-forming structure may comprise an element that surrounds a mouth region in use, e.g., by forming a seal on a lower lip region of the face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g., because of the different shape, structure, variability and sensitivity of different regions of the patient's face. For example, a structure of swimming goggles that overlays a patient's forehead to seal therewith may not be appropriate to use on a patient's nose.

Certain seal-forming structures may be designed for mass manufacture such that one design can fit and be comfortable and effective for a wide range of different face shapes and sizes. To the extent to which there is a mismatch between the shape of the patient's face, and the seal-forming structure of the mass-manufactured patient interface, one or both must adapt in order for the seal-forming structure seal with the patient's face.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force may be required to position the patient interface against the face in order to seal therewith or leak may occur. Additional force may cause discomfort for the patient during use.

Another type of seal-forming structure incorporates a flap of relatively thin material positioned about the periphery of the mask so as to provide a self-sealing action against the face of the patient when positive pressure is applied within the mask. Like the type of seal-forming structure described in the preceding paragraph, if the size and shape of the seal-forming structure does not accurately correspond to the size and shape of the patient's face, additional force may be required to seal against the patient's face, or the mask may leak. Additional force may cause discomfort for the patient during use. Furthermore, if the size and shape of the seal-forming structure does not accurately correspond to that of the patient, then the seal-forming structure may crease or buckle in use due its relative thinness, which may result in leaking.

Another type of seal-forming structure may comprise a friction-fit element, e.g., for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited, Kwok et al., International Publication No. WO 1998/004310 A1; Davidson et al., International Publication No.WO 2006/074513 A1; Dravitzki et al., International Publication No. WO 2010/135785 A1.

One form of nasal pillow is found in the ADAM Circuit manufactured by Puritan-Bennett Corporation. Another nasal pillow, or nasal puff is the subject of Trimble et al., U.S. Patent No. 4,782,832, assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: Gunaratnam et al., International Publication No. WO 2004/073778 A1 (describing amongst other things aspects of the ResMed Limited SWIFT^{™} nasal pillows); Guney et al., U.S. Publication No. 2009/0044808 A1 (describing amongst other things aspects of the ResMed Limited SWIFT^{™} LT nasal pillows); Davidson et al., International Publication No. WO 2005/063328 A1 and Lubke et al., International Publication No. WO 2006/130903 A1 (describing amongst other things aspects of the ResMed Limited MIRAGE LIBERTY^{™} full-face mask); Rummery et al., International Publication No. WO 2009/052560 A1 (describing amongst other things aspects of the ResMed Limited SWIFT^{™} FX nasal pillows).

### 2.2.3.1.2 Positioning and stabilising

A seal-forming structure of a patient interface used for positive air pressure therapy is subject to the corresponding force of the air pressure to disrupt a seal. Thus, a variety of techniques have been used to position the seal-forming structure, and to maintain it in sealing relation with the appropriate portion of the face.

One technique is the use of adhesives. See, for example, Kooij et al., U.S. Publication No. 2010/0000534 A1. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus, RPT devices may also act as flow therapy devices. Examples of RPT devices include a CPAP device and a ventilator.

Air pressure generators are known in a range of applications, e.g., industrial-scale ventilation systems. However, air pressure generators for medical applications have particular requirements not fulfilled by more generalised air pressure generators, such as the reliability, size, and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is minimization of acoustic noise because the patient may be sleeping during operation.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series TANGO^{™} | 31.9 | 2007 |
| C-Series TANGO^{™} with Humidifier | 33.1 | 2007 |
| S8 ESCAPE^{™} II | 30.5 | 2005 |
| S8 ESCAPE^{™} II with H4i^{™} Humidifier | 31.1 | 2005 |
| S9 AUTOSET^{™} | 26.5 | 2010 |
| S9 AUTOSET^{™} with H5i^{™} Humidifier | 28.6 | 2010 |

One known RPT device used for treating sleep disordered breathing is the S9^{™} Sleep Therapy System, manufactured by ResMed Limited. Another example of an RPT device is a ventilator. Ventilators such as the ResMed STELLAR^{™} Series of Adult and Paediatric Ventilators may provide support for invasive and non-invasive non-dependent ventilation for a range of patients for treating a number of conditions such as but not limited to NMD, OHS and COPD.

The ELISÉE^{™} 150 ventilator and VS III^{™} ventilator, manufactured by ResMed Limited, may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, warm air applied generally to the face area in and about the patient interface in cooler climates is more comfortable than cold air. Humidifiers therefore often have the capacity to heat the flow of air was well as humidifying it. Moreover, warmer air has a greater capacity for water vapor.

A range of artificial humidification devices and systems are known, however, they may not fulfil the specialised requirements of a medical humidifier.

Medical humidifiers are used to increase humidity and/or temperature of the flow of air in relation to ambient air when required, typically where the patient may be asleep or resting (e.g., at a hospital). A medical humidifier for bedside placement may be small. A medical humidifier may be configured to only humidify and/or heat the flow of air delivered to the patient without humidifying and/or heating the patient's surroundings. Room-based systems (e.g., a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, and some are difficult or inconvenient to use by patients.

### 2.2.3.5 Data Management

There may be clinical reasons to obtain data to determine whether the patient prescribed with respiratory therapy has been "compliant", e.g., that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.6 Vent technologies

Some forms of treatment systems may include a vent to allow the washout of exhaled carbon dioxide. The vent may allow a flow of gas from an interior space of a patient interface, e.g., from within the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g., through noise or focussed airflow.

ResMed Limited has developed a number of improved mask vent technologies. *See* Kwok, International Publication No. WO 1998/034665 A1; Gunaratnam et al., International Publication No. WO 2000/078381 A1; Drew et al., U.S. Patent No. 6,581,594 B1; Ng et al., U.S. Publication No. 2009/0050156 A1; Guney et al., U.S. Publication No. 2009/0044808 A1.

Table of noise of prior masks (ISO 17510-2:2007, 10 cmH₂O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| RESMED^{™} MIRAGE^{™} (*) | nasal | 29.5 | 21.5 | 1998 |
| RESMED^{™} ULTRA MIRAGE^{™} | nasal | 36 (3) | 28 (3) | 2000 |
| RESMED^{™} MIRAGE ACTIVA^{™} | nasal | 32 (3) | 24 (3) | 2002 |
| RESMED^{™} MIRAGE MICRO^{™} | nasal | 30 (3) | 22 (3) | 2008 |
| RESMED^{™} MIRAGE^{™} SoftGel | nasal | 29 (3) | 22 (3) | 2008 |
| RESMED^{™} MIRAGE^{™} FX | nasal | 26 (3) | 18 (3) | 2010 |
| RESMED^{™} MIRAGE SWIFT^{™} (*) | nasal pillows | 37 | 29 | 2004 |
| RESMED^{™} MIRAGE SWIFT^{™} II | nasal pillows | 28 (3) | 20 (3) | 2005 |
| RESMED^{™} MIRAGE SWIFT^{™} LT | nasal pillows | 25 (3) | 17 (3) | 2008 |
| RESMED^{™} AIRFIT^{™} P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2.2.3.7 Heat and Moisture Exchanger (HMX) technology

A patient may be susceptible to drying of the internal airways passages while undergoing various forms of respiratory therapy as described above. For example, CPAP therapy entails providing the patient with a flow of air pressurized at a pressure greater than ambient continuously, and this continuous flow of air, particularly at an elevated level in conjunction with the positive air pressure, may causing drying of the patient's airways. That drying may cause discomfort, which in turn may negatively impact the patient's compliance with therapy.

To minimize the drying effect of these forms of respiratory therapy, the flow of air provided to the patient may be humidified before it reaches the patient. Certain forms of humidification technology, such as those described above, actively provide humidified air to the patient to reduce the drying effect by heating a reservoir of water and passing air over its surface to increase the absolute humidity of the air, *i.e.,* the air absorbs water vapour from the reservoir. The humidified air is then passed to the patient via the air circuit. The air circuit may also be heated to prevent condensation, also known as rainout, of the water vapour within the air circuit during transport to the patient. These forms of technology typically involve filling the reservoir with water before therapy, and then the reservoir is provided to the RPT system so that the water can be heated to humidify the air for therapy. The reservoir typically requires regular cleaning, there is a risk of spillage, which may be particularly problematic in the context of electrical components, and the reservoir requires refilling by the patient before use.

Eliminating the need for a pre-supplied water source, such as a water-filled reservoir, and input electrical power to heat the water may provide several benefits. For example, the RPT device could be made smaller because it would not require space for the water reservoir and heating plate. Since no electrical energy is consumed in heating of the water, electricity costs may be reduced. Also, fewer electrical components may be needed in the RPT device, which reduces its cost and complexity. Also, the RPT device may be easier to use because there is no water reservoir to fill, empty, and clean. Risk of spillage may be reduced as well. Also, operation of the RPT device may be simplified because there are no humidification settings to operate.

In operation, the patient breathes out (exhalation) air that has been heated within the patient's body and that has absorbed water vapour from the patient's airways. The heat and moisture in the exhaled air is captured by the HMX material(s), *i.e.,* the HMX material(s) are heated by the relatively warm exhaled air and the HMX material(s) adsorb water vapour from the relatively humid exhaled air, as the exhaled air passes through HMX material(s) prior to being vented to atmosphere. During inhalation, the flow of pressurized air passes through the HMX material(s) in the opposite direction to exhalation to reach the patient's airways, and the source of the incoming air is typically ambient air. Thus, the flow of pressurized air, as it passes through the HMX material(s) prior to reaching the patient's airways, absorbs moisture in the form of water vapour as it is desorbed from the HMX material(s) and the flow of pressurized air is heated by heat released from the HMX material(s).

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of the present technology is directed to a patient interface that may comprise: a plenum chamber; a seal-forming structure; and a positioning and stabilising structure. The patient interface may further comprise a vent structure. The patient interface may further be configured to leave the patient's mouth uncovered, or if the seal-forming structure is configured to seal around the patient's nose and mouth, the patient interface may be further configured to allow the patient to breath from ambient in the absence of a flow of pressurised air through the plenum chamber inlet port.

Another aspect of the present technology is directed to a patient interface comprising: a plenum chamber pressurisable to a therapeutic pressure of at least 4 cmH2O above ambient air pressure, the plenum chamber including a plenum chamber inlet port sized and structured to receive a flow of air at the therapeutic pressure for breathing by a patient; a seal-forming structure constructed and arranged to seal with a region of the patient's face surrounding an entrance to the patient's airways, the seal-forming structure having a hole therein such that the flow of air at the therapeutic pressure is delivered to at least an entrance to the patient's nares, the seal-forming structure constructed and arranged to maintain the therapeutic pressure in the plenum chamber throughout the patient's respiratory cycle in use; a positioning and stabilising structure configured to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use; and a vent structure configured to allow a continuous flow of gases exhaled by the patient from an interior of the plenum chamber to ambient, the vent structure being sized and shaped to maintain the therapeutic pressure in the plenum chamber in use; wherein the patient interface is configured to leave the patient's mouth uncovered, or if the seal-forming structure is configured to seal around the patient's nose and mouth, the patient interface is configured to allow the patient to breath from ambient in the absence of a flow of pressurised air through the plenum chamber inlet port.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising two plenum chamber connectors; a seal-forming structure being constructed and arranged to seal with a region of the patient's face that at least partly surrounds an entrance to the patient's airways; a positioning and stabilising structure configured to hold the seal-forming structure in a therapeutically effective position on the patient's head, the positioning and stabilising structure further comprising two conduits being configured to connect to a corresponding one of the plenum chamber connectors; a vent assembly having a plurality of vent holes configured to allow a continuous flow of gases exhaled by the patient from within the plenum chamber to ambient throughout the patient's respiratory cycle; and a heat and moisture exchanger (HMX) assembly including an HMX frame and a material, the material being configured to adsorb water vapour from gases exhaled by the patient and desorb water vapour into the flow of air at the therapeutic pressure, the material being supported by the HMX frame within the plenum chamber such that at least a portion of the flow of air at the therapeutic pressure entering the plenum chamber from the plenum chamber connectors passes through the material before entering the patient's airways.

Another aspect of the present technology is directed to a patient interface comprising: a plenum chamber pressurisable to a therapeutic pressure of at least 4 cmH2O above ambient air pressure by a flow of air at the therapeutic pressure for breathing by a patient, the plenum chamber further comprising two plenum chamber connectors, each of the plenum chamber connectors being positioned on a corresponding lateral side of the plenum chamber and configured to receive the flow of air at the therapeutic pressure; a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use; a positioning and stabilising structure configured to hold the seal-forming structure in a therapeutically effective position on the patient's head, the positioning and stabilising structure further comprising two conduits, each of the conduits being configured to be positioned on a corresponding lateral side of the patient's head in use and being configured to connect to a corresponding one of the plenum chamber connectors; a vent assembly connected to the plenum chamber and having a plurality of vent holes configured to allow a continuous flow of gases exhaled by the patient from within the plenum chamber to ambient throughout the patient's respiratory cycle, the vent holes being sized and shaped to maintain the therapeutic pressure in the plenum chamber in use; and a heat and moisture exchanger (HMX) assembly connected to the vent assembly, the HMX assembly including an HMX frame and a material positioned on the HMX frame, the material being configured to adsorb water vapour from gases exhaled by the patient and desorb water vapour into the flow of air at the therapeutic pressure, the material being supported by the HMX frame within the plenum chamber such that at least a portion of the flow of air at the therapeutic pressure entering the plenum chamber from the plenum chamber connectors passes through the material before entering the patient's airways; wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.

In examples of any of the aspects described in the preceding paragraphs: (a) the material may comprise foam or paper, (b) the material may comprise a salt applied to a surface of the material, (c) the material may be removable from the HMX frame, (d) the HMX assembly may be removable from within the plenum chamber, (e) the vent assembly may be removably connected to the plenum chamber, (f) the vent assembly and the HMX assembly may be removable together from the plenum chamber, (g) the HMX assembly may be removable from the vent assembly, (h) the vent assembly may comprise a vent body and a vent cap attached to the vent body, (i) the vent holes may be formed between the vent body and the vent cap, (j) the vent body may comprise vent body holes configured to allow the continuous flow of gases exhaled by the patient to pass through the vent body and then to atmosphere through the vent holes, (k) the vent assembly may include a vent diffuser material positioned between the vent body and the vent cap, (1) the vent body may include a spacer to space the vent diffuser material away from the vent body holes and against the vent cap, (m) one of the plenum chamber and the vent body may include one or more orientation indication protrusions, and the other of the plenum chamber and the vent body may include one or more orientation indication recesses configured to receive corresponding ones of the orientation indication protrusions when the vent body is attached to the plenum chamber, (n) one of the plenum chamber and the vent body may include a connection lip, and the other of the plenum chamber and the vent body may include a connection channel configured to receive the connection lip to removably attach the vent body to the plenum chamber, (o) the plenum chamber may include a receiving hole configured to receive the vent assembly and through which the HMX assembly is inserted inside of the plenum chamber, (p) the HMX frame may be constructed from a flexible material, (q) the flexible material may be silicone rubber or thermoplastic elastomer, (r) the material may be positioned within the plenum chamber such that the flow of air at the therapeutic pressure must pass through the material before entering the patient's airways, (q) the material may be positioned within the plenum chamber such that gas exhaled by the patient must pass through the material before exiting to ambient through the vent holes, (r) the material may be positioned within the plenum chamber such that at least a portion of the flow of air at the therapeutic pressure must bypasses the material before entering the patient's airways, (s) the material may be positioned within the plenum chamber such that at least a portion of the gas exhaled by the patient bypasses the material before exiting to ambient through the vent holes, (t) the HMX frame may form an HMX frame hole, and a peripheral portion of the material may be positioned on the HMX frame such that gases flowing through the material pass through the HMX frame hole, (u) the HMX frame may include an HMX rim connected to the vent assembly, (v) the HMX frame may include one or more HMX struts joined to the HMX rim, (w) each of the HMX struts may be straight, (x) each of the HMX struts may be bent, the HMX rim may be joined directly to the HMX frame, (y) the vent body may include an attachment structure configured to receive the HMX rim to attach the HMX frame to the vent body, (z) one or more slots may be formed through the attachment structure, each of the slots being configured to receive a corresponding one of the HMX struts, (aa) the material may be permanently attached to the HMX frame, (bb) the material may be attached to the HMX frame by one or more of the following: an adhesive, overmoulding the HMX frame onto the material, and retainers to hold the material against the HMX frame, (cc) the material may comprise a posterior surface that faces the patient's face in use, the posterior surface being positively curved to avoid contact with the patient's face during use, (dd) the seal-forming structure may comprise a sealing lip configured to seal against the HMX frame, (ee) the sealing lip may include a sealing lip bypass opening configured to allow at least a portion of the flow of air at the therapeutic pressure to bypass the material before entering the patient's airways and to allow at least a portion of the gas exhaled by the patient to bypass the material before exiting to ambient through the vent holes, (ff) the sealing lip may be configured to seal around the entire perimeter of the HMX frame such that all of the flow of air at the therapeutic pressure passes through the material before entering the patient's airways and all of the gas exhaled by the patient passes through the material before exiting to ambient through the vent holes, (gg) the seal-forming structure may be configured to leave the patient's mouth uncovered and the seal-forming structure further comprises nasal pillows or a nasal cradle, and/or (hh) the seal-forming structure may be configured to seal around the patient's nose and mouth and the seal-forming structure further comprises a nasal hole or two nasal holes that are configured to pneumatically communicate with the patient's nares in use and a mouth hole that is configured to pneumatically communicate with the patient's mouth in use.

Another aspect of the present technology is directed to a respiratory pressure therapy (RPT) system comprising: the patient interface of any one of the aspects and examples described in the preceding paragraphs; a respiratory pressure therapy (RPT) device configured to generate the flow of air at the therapeutic pressure; and an air circuit configured to connect the RPT device and the patient interface to direct the flow of air at the therapeutic pressure from the RPT device to the patient interface. In a further example, the RPT system may not include a humidifier.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1 shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.
Fig. 2 shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

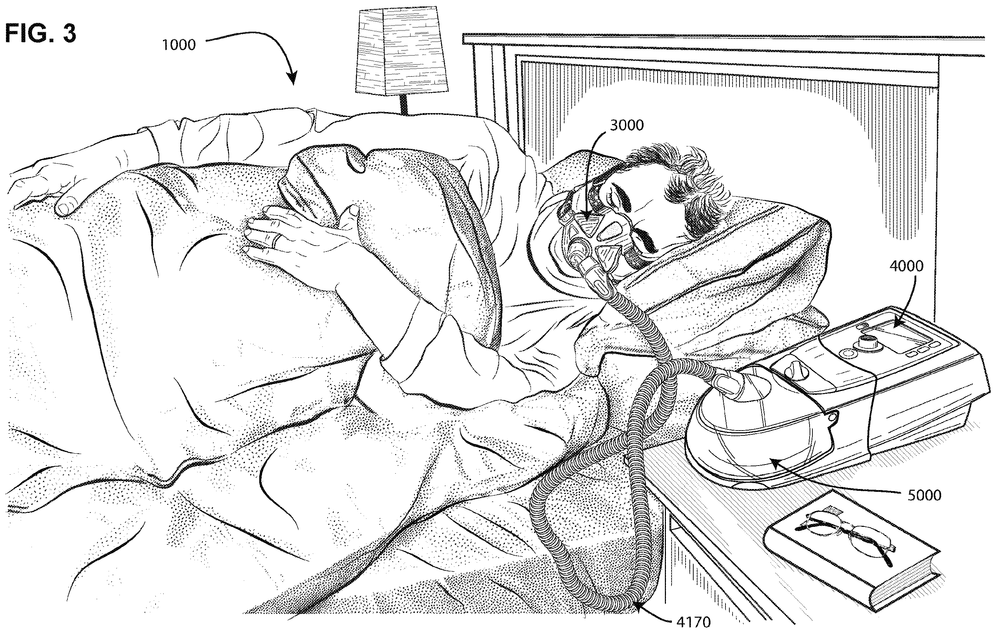

Fig. 3 shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

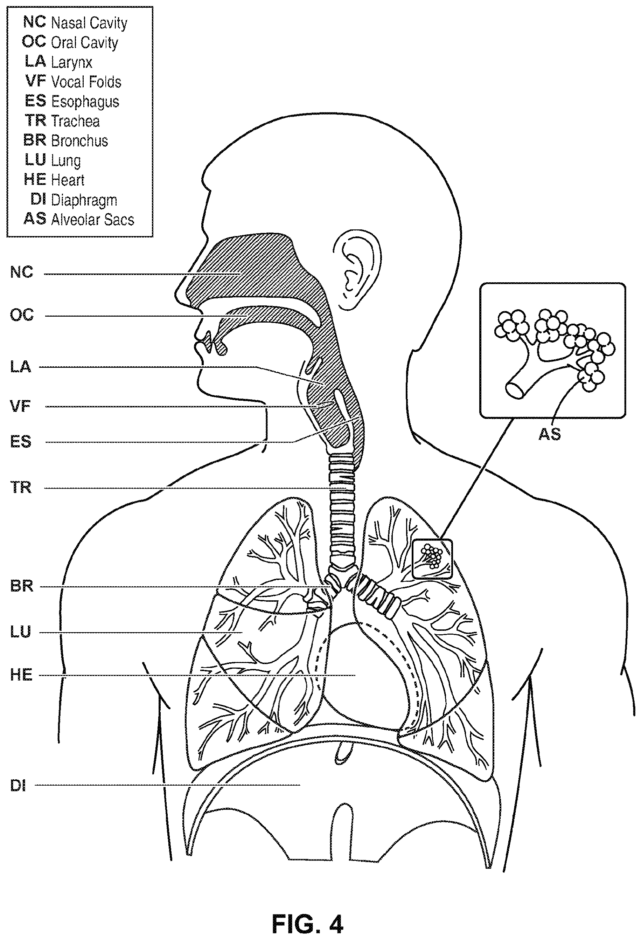

Fig. 4 shows an overview of a human respiratory system including the nasal and oral cavities, the larynx, vocal folds, oesophagus, trachea, bronchus, lung, alveolar sacs, heart and diaphragm.

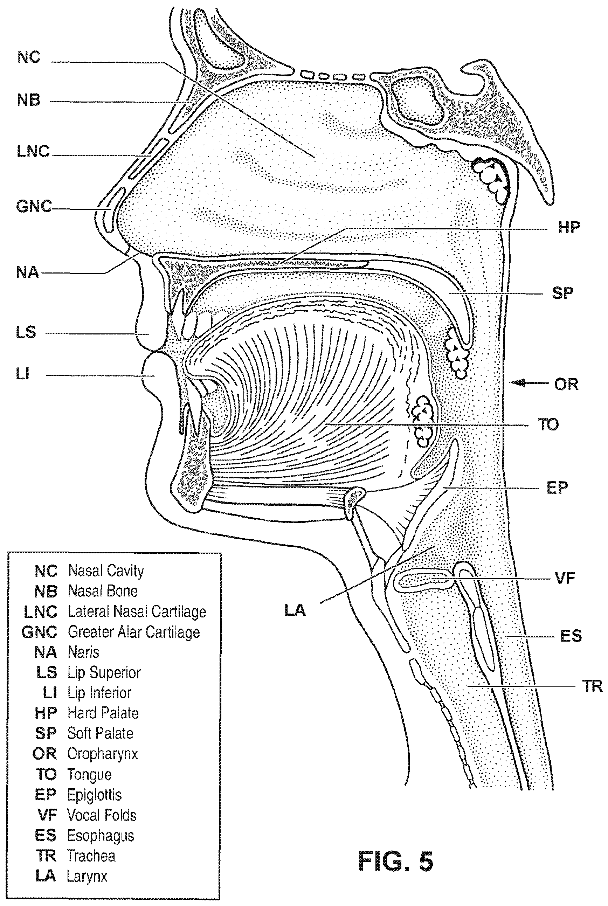

Fig. 5 shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

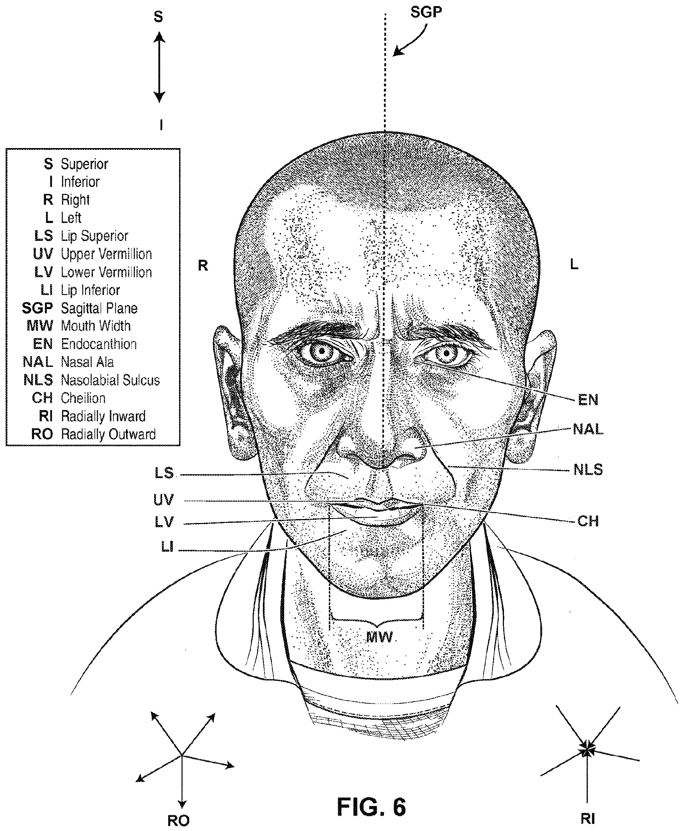

Fig. 6 is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

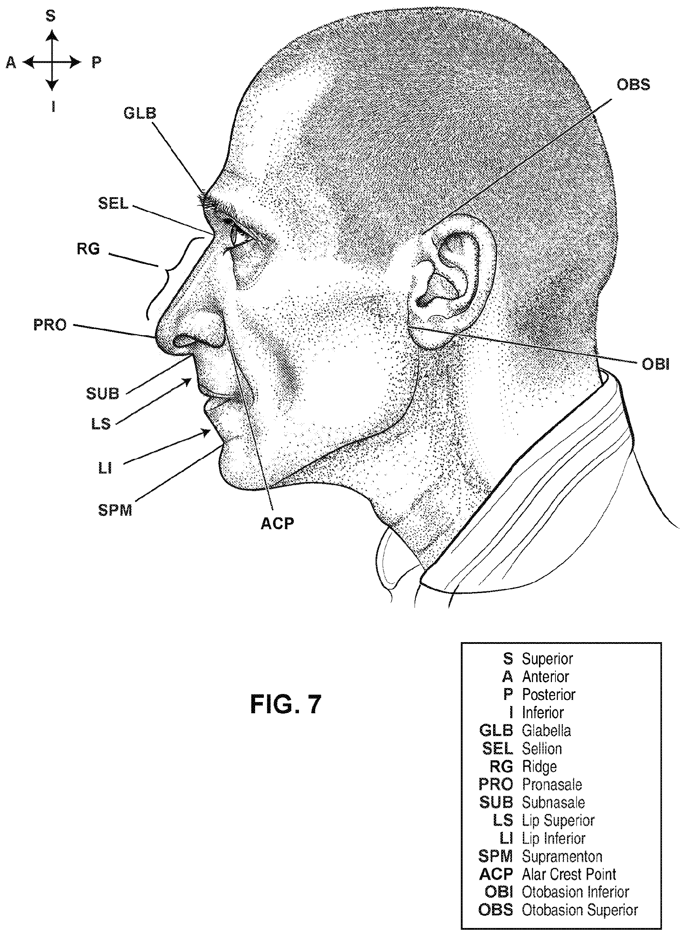

Fig. 7 is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

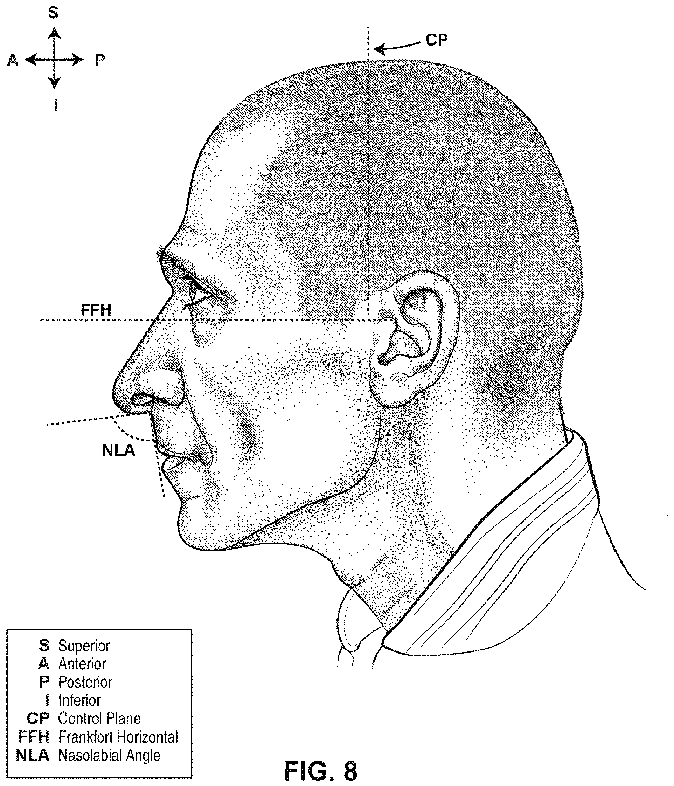

Fig. 8 is a further side view of a head. The approximate locations of the Frankfort horizontal and nasolabial angle are indicated. The coronal plane is also indicated.

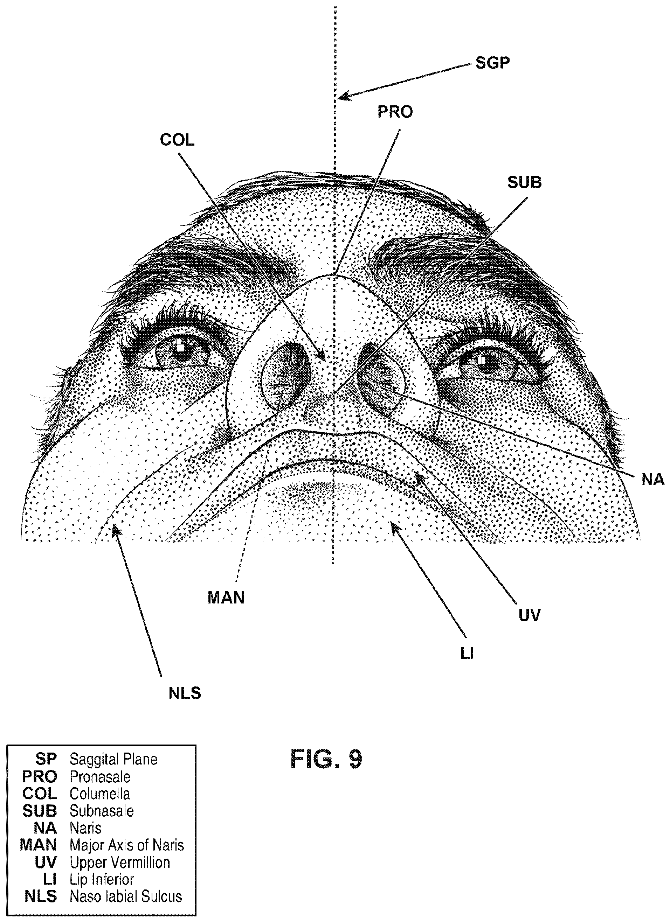

Fig. 9 shows a base view of a nose with several features identified including naso-labial sulcus, lip inferior, upper Vermilion, naris, subnasale, columella, pronasale, the major axis of a naris and the midsagittal plane.

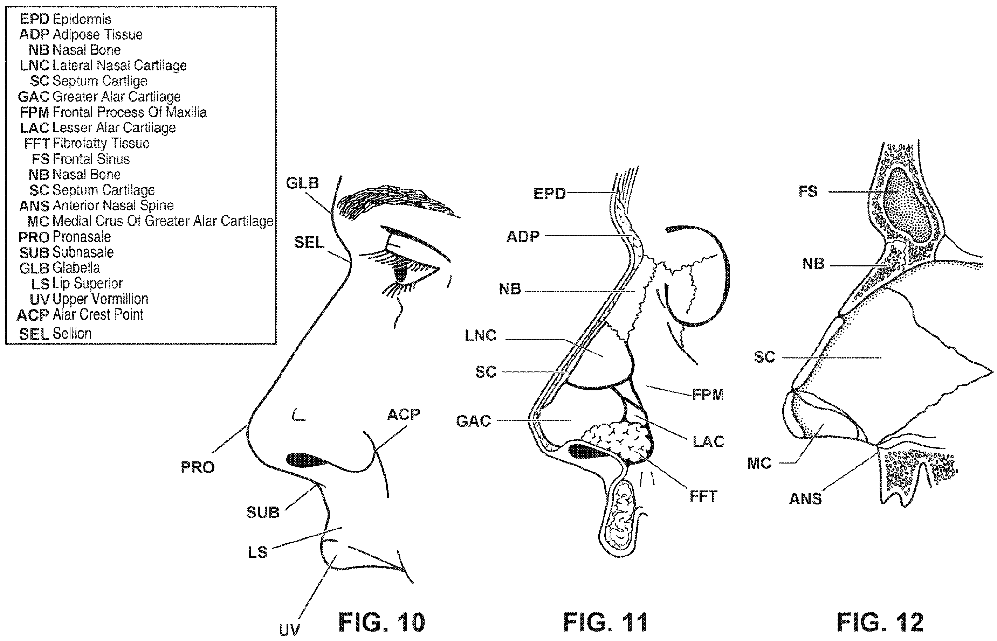

Fig. 10 shows a side view of the superficial features of a nose.
Fig. 11 shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 12 shows a medial dissection of a nose, approximately several millimeters from the midsagittal plane, amongst other things showing the septum cartilage and medial crus of greater alar cartilage.

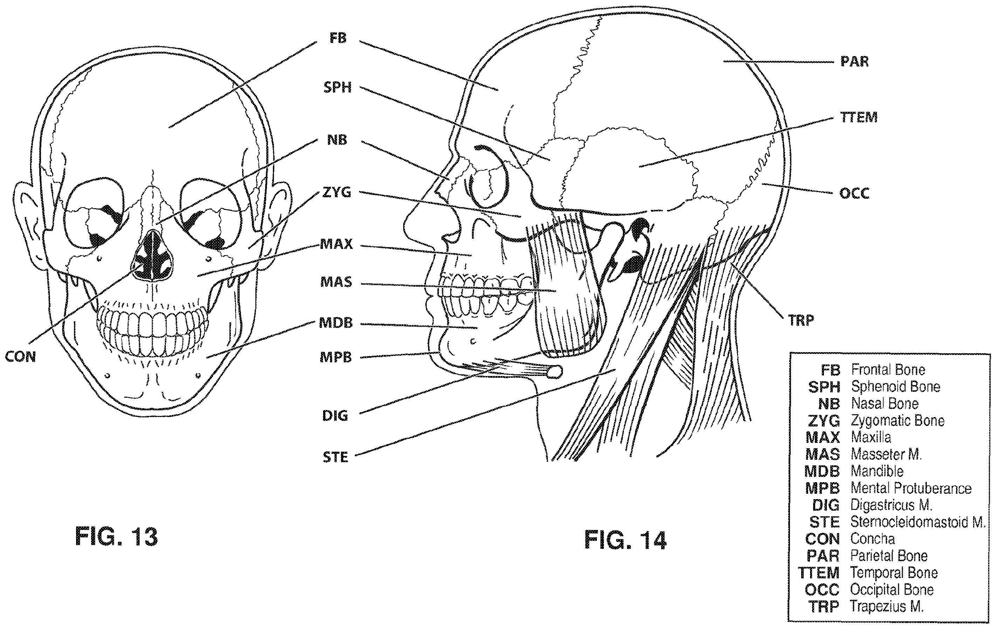

Fig. 13 shows a front view of the bones of a skull including the frontal, nasal and zygomatic bones. Nasal concha are indicated, as are the maxilla, and mandible.
Fig. 14 shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

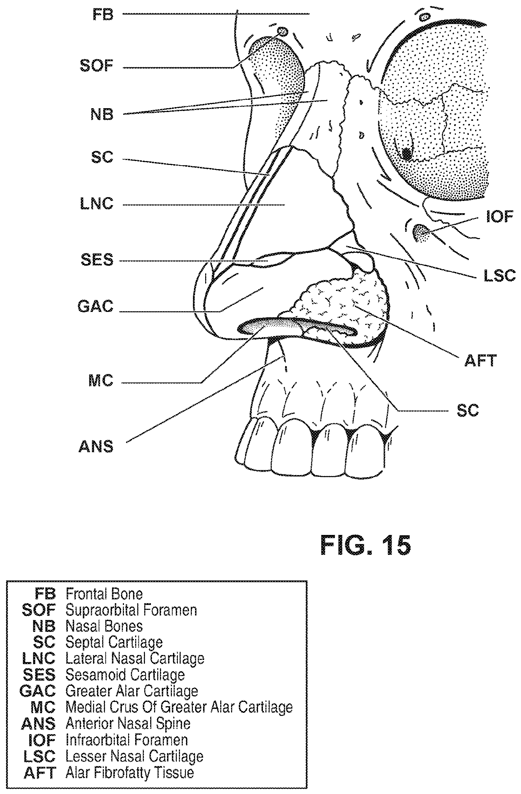

Fig. 15 shows an anterolateral view of a nose.

### 4.3 PATIENT INTERFACE

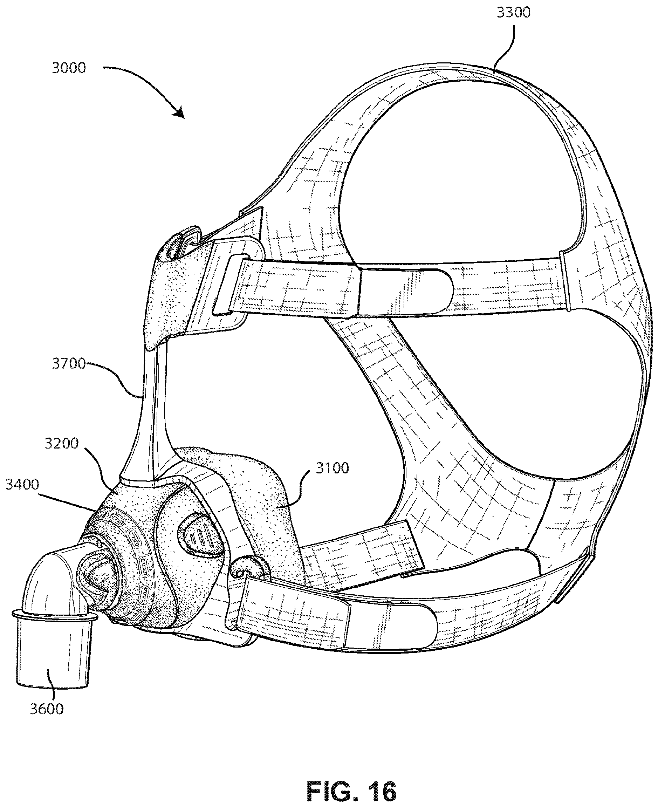

Fig. 16 shows a patient interface in the form of a nasal mask in accordance with one form of the present technology.
Fig. 17 shows a schematic of a cross-section through a structure at a point. An outward normal at the point is indicated. The curvature at the point has a positive sign, and a relatively large magnitude when compared to the magnitude of the curvature shown in Fig. 18.
Fig. 18 shows a schematic of a cross-section through a structure at a point. An outward normal at the point is indicated. The curvature at the point has a positive sign, and a relatively small magnitude when compared to the magnitude of the curvature shown in Fig. 17.
Fig. 19 shows a schematic of a cross-section through a structure at a point. An outward normal at the point is indicated. The curvature at the point has a value of zero.
Fig. 20 shows a schematic of a cross-section through a structure at a point. An outward normal at the point is indicated. The curvature at the point has a negative sign, and a relatively small magnitude when compared to the magnitude of the curvature shown in Fig. 21.
Fig. 21 shows a schematic of a cross-section through a structure at a point. An outward normal at the point is indicated. The curvature at the point has a negative sign, and a relatively large magnitude when compared to the magnitude of the curvature shown in Fig. 20.

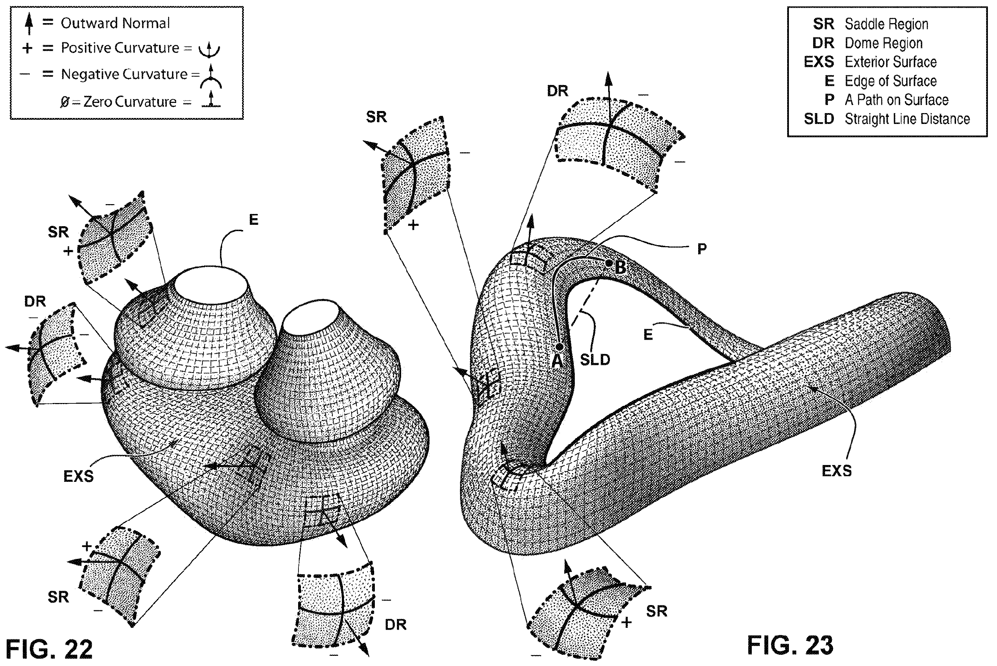

Fig. 22 shows a cushion for a mask that includes two pillows. An exterior surface of the cushion is indicated. An edge of the surface is indicated. Dome and saddle regions are indicated.
Fig. 23 shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 24 shows the surface of a structure, with a one dimensional hole in the surface. The illustrated plane curve forms the boundary of a one dimensional hole.
Fig. 25 shows a cross-section through the structure of Fig.24. The illustrated surface bounds a two dimensional hole in the structure of Fig. 24.
Fig. 26 shows a perspective view of the structure of Fig. 24, including the two dimensional hole and the one dimensional hole. Also shown is the surface that bounds a two dimensional hole in the structure of Fig. 24.
Fig. 27 shows a mask having an inflatable bladder as a cushion.
Fig. 28 shows a cross-section through the mask of Fig. 27, and shows the interior surface of the bladder. The interior surface bounds the two dimensional hole in the mask.
Fig. 29 shows a further cross-section through the mask of Fig. 27. The interior surface is also indicated.
Fig. 30 illustrates a left-hand rule.
Fig. 31 illustrates a right-hand rule.
Fig. 32 shows a left ear, including the left ear helix.
Fig. 33 shows a right ear, including the right ear helix.
Fig. 34 shows a right-hand helix.
Fig. 35 shows a view of a mask, including the sign of the torsion of the space curve defined by the edge of the sealing membrane in different regions of the mask.
Fig. 36 shows a view of a plenum chamber 3200 showing a sagittal plane and a mid-contact plane.
Fig. 37 shows a view of a posterior of the plenum chamber of Fig. 36. The direction of the view is normal to the mid-contact plane. The sagittal plane in Fig. 37 bisects the plenum chamber into left-hand and right-hand sides.

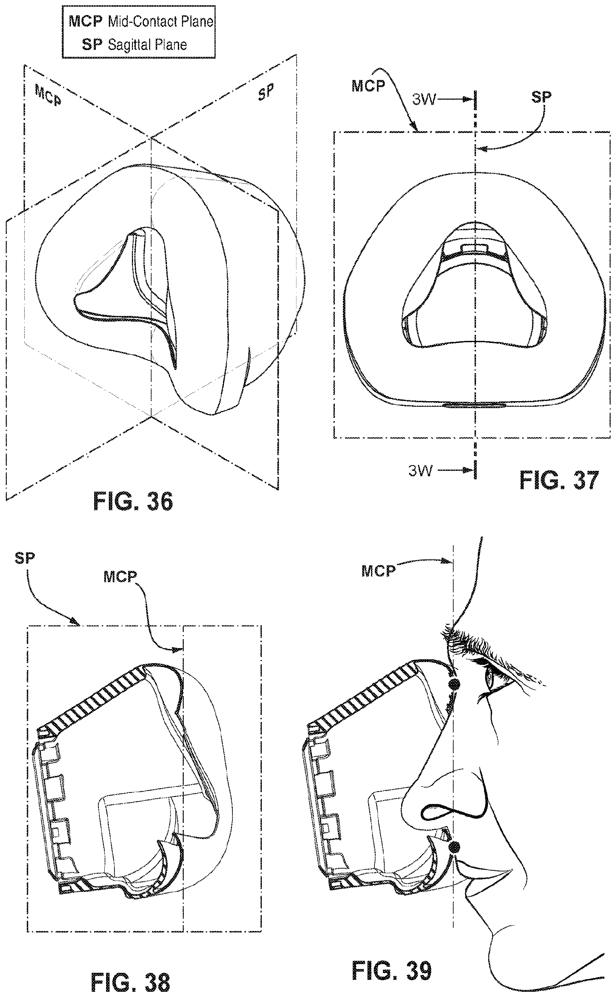

Fig. 38 shows a cross-section through the plenum chamber of Fig. 37, the cross-section being taken at the sagittal plane shown in Fig. 37. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 38 shows the plenum chamber 3200 of Fig. 36 in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 38 the plenum chamber 3200 is that of a nasal mask, and the superior point 3220 sits approximately on the sellion, while the inferior point 3230 sits on the lip superior.

### 4.4 RPT DEVICE

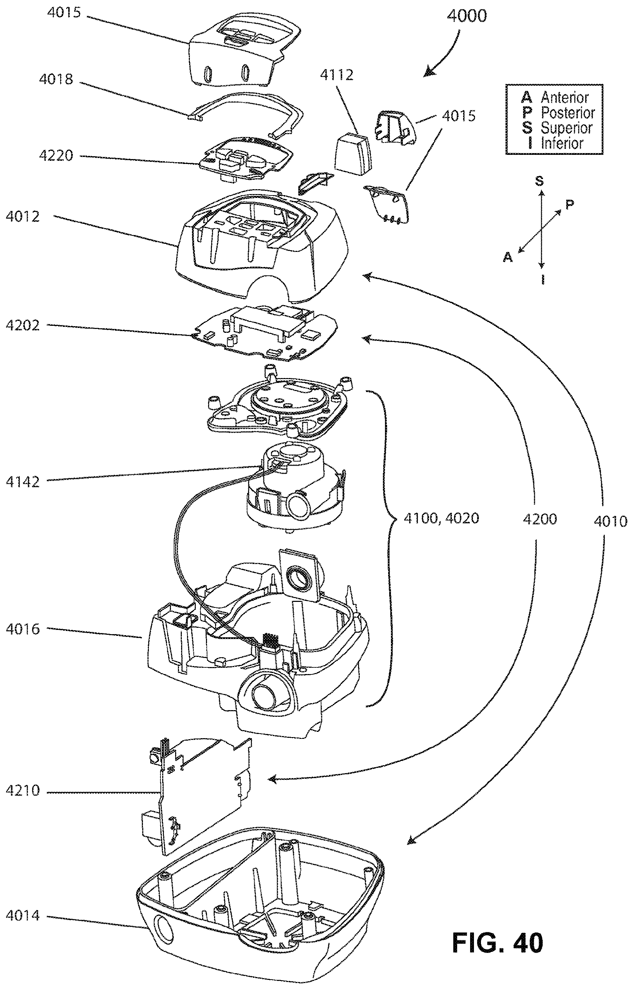

Fig. 40 shows an RPT device in accordance with one form of the present technology.

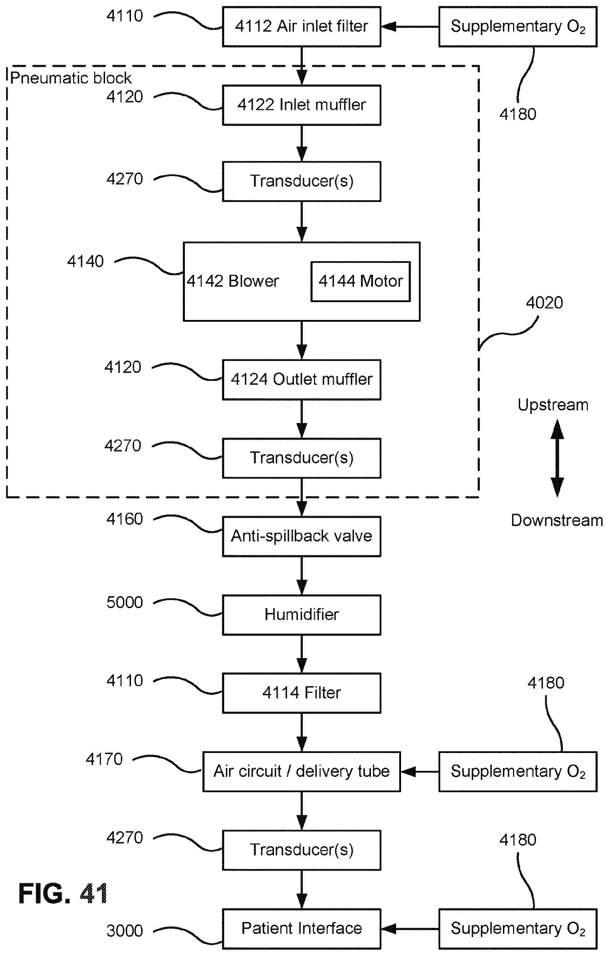

Fig. 41 is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 BREATHING WAVEFORMS

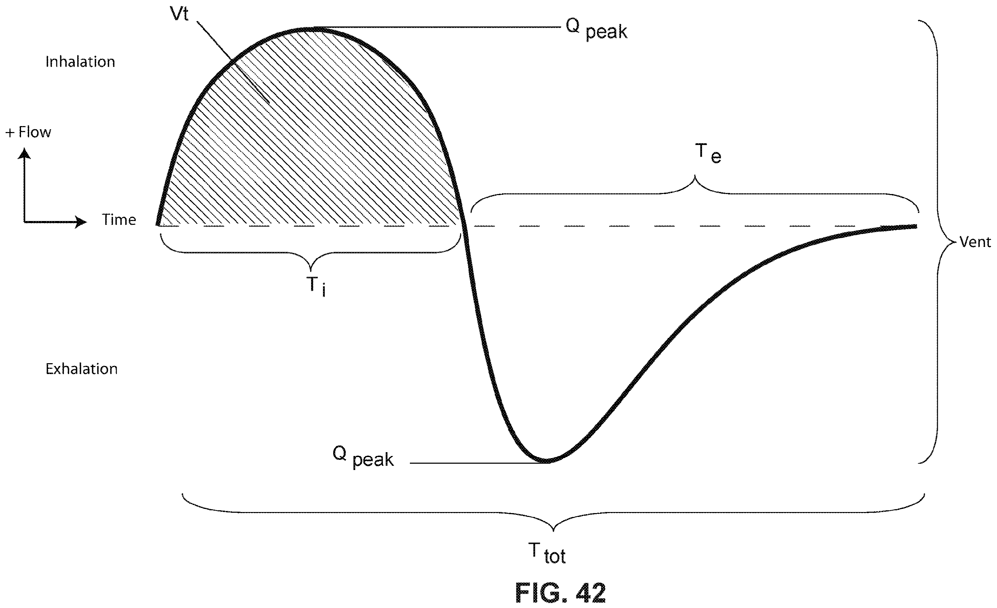

Fig. 42 shows a model typical breath waveform of a person while sleeping.

### 4.6 PATIENT INTERFACE OF THE PRESENT TECHNOLOGY

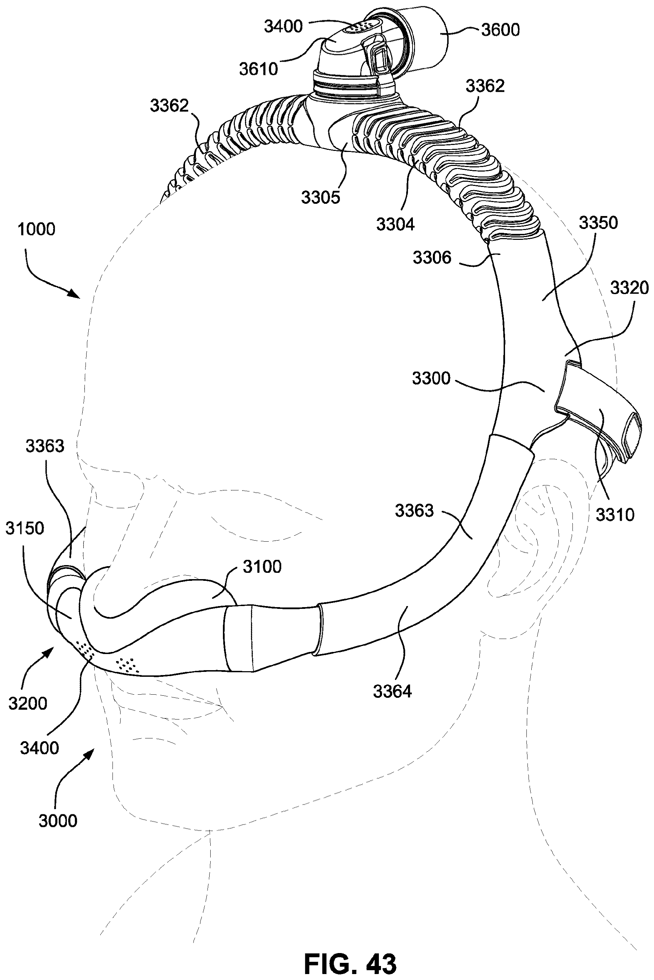

Fig. 43 shows a perspective view of a patient interface according to an example of the present technology on a patient.

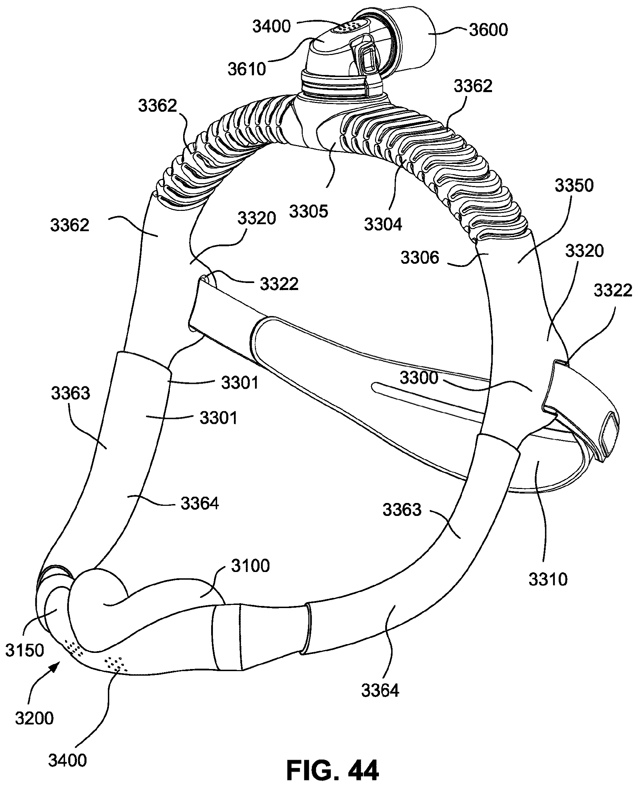

Fig. 44 shows a perspective view of a patient interface according to an example of the present technology.

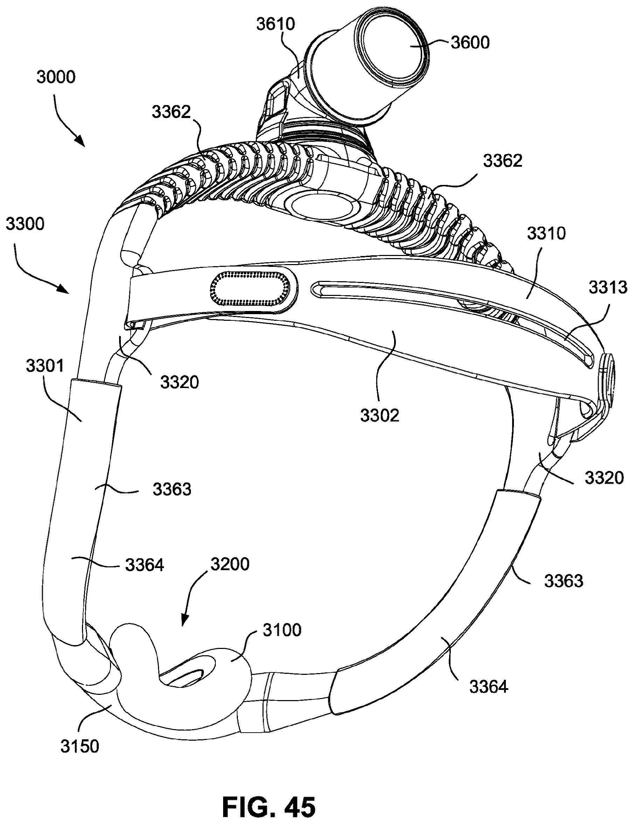

Fig. 45 shows a rear perspective view of a patient interface according to an example of the present technology.

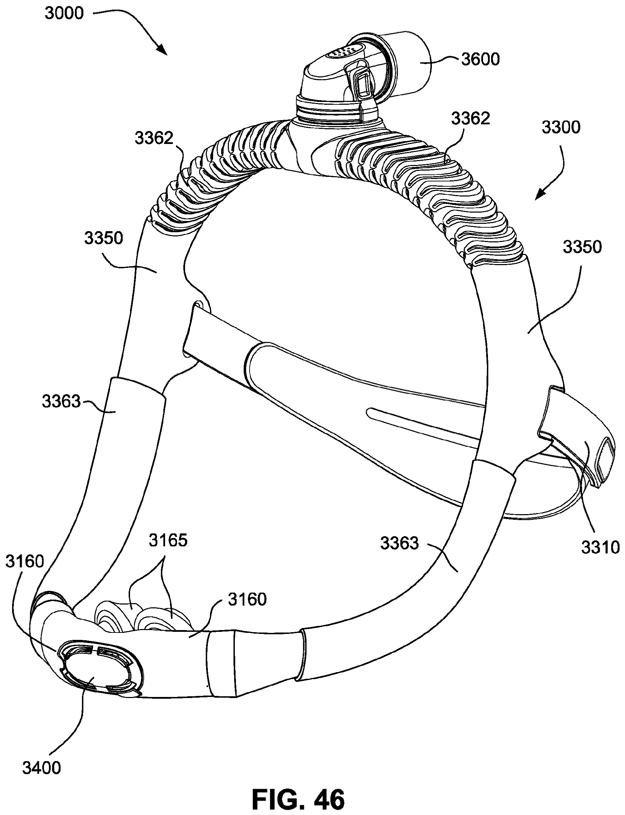

Fig. 46 shows a perspective view of a patient interface according to another example of the present technology.

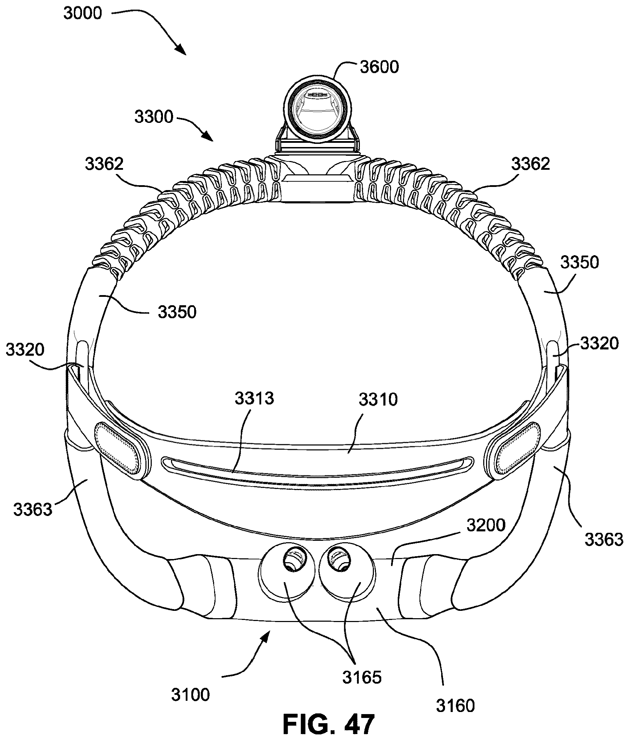

Fig. 47 shows a rear view of a patient interface according to another example of the present technology.

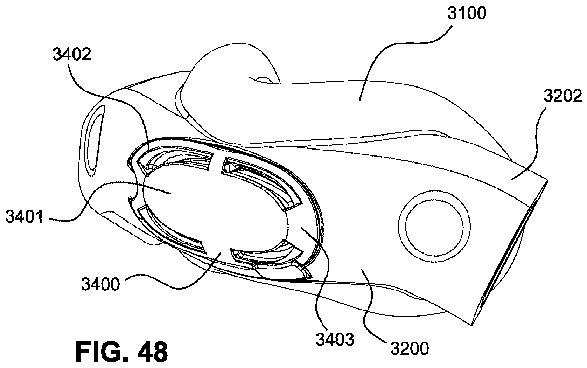

Fig. 48 shows a perspective view of a seal-forming structure, a plenum chamber, and a vent of a patient interface according to another example of the present technology.

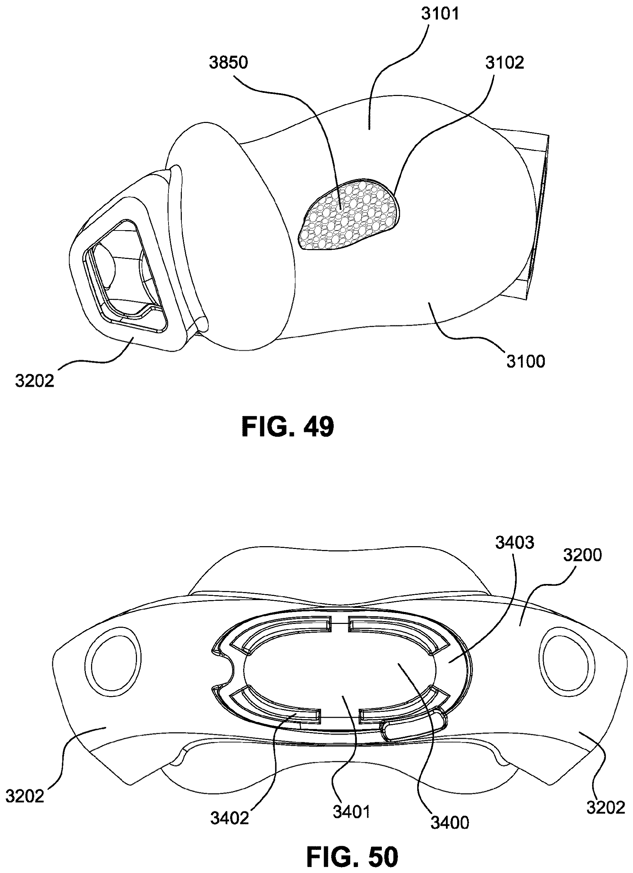

Fig. 49 shows a rear perspective view of a seal-forming structure and a plenum chamber of a patient interface according to another example of the present technology.
Fig. 50 shows a front view of a seal-forming structure, a plenum chamber, and a vent of a patient interface according to another example of the present technology.

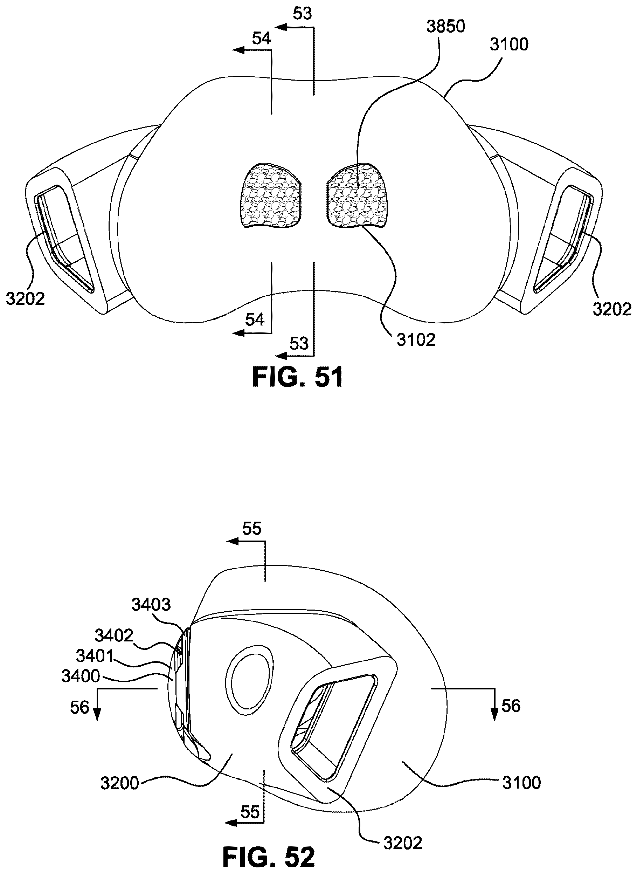

Fig. 51 shows a rear view of a seal-forming structure and a plenum chamber of a patient interface according to another example of the present technology.
Fig. 52 shows a side view of a seal-forming structure, a plenum chamber, and a vent of a patient interface according to another example of the present technology.

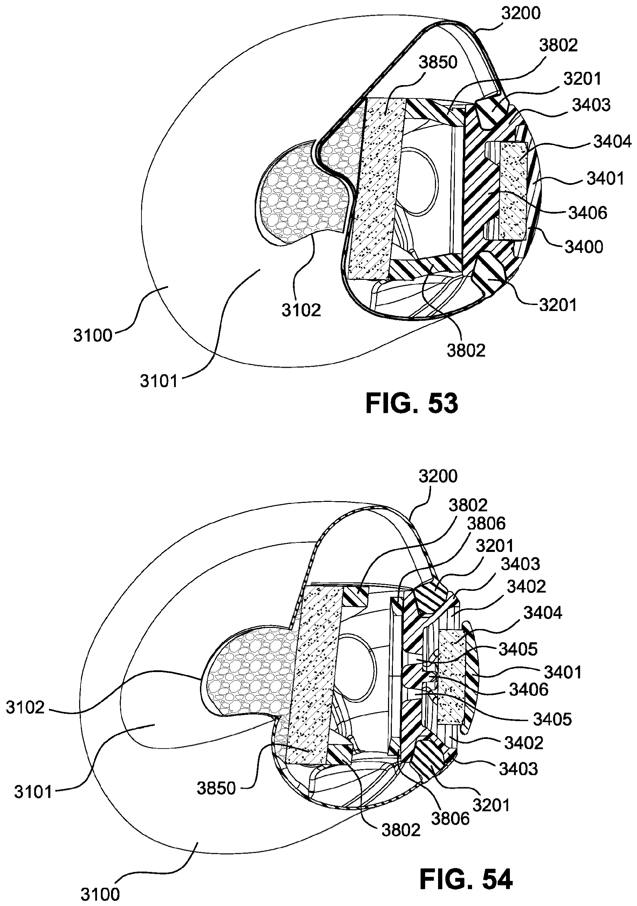

Fig. 53 shows a cross-sectional view taken through line 53-53 of Fig. 51 of a seal-forming structure, a plenum chamber, and a vent of a patient interface according to another example of the present technology.
Fig. 54 shows a cross-sectional view taken through line 54-54 of Fig. 51 of a seal-forming structure, a plenum chamber, and a vent of a patient interface according to another example of the present technology.

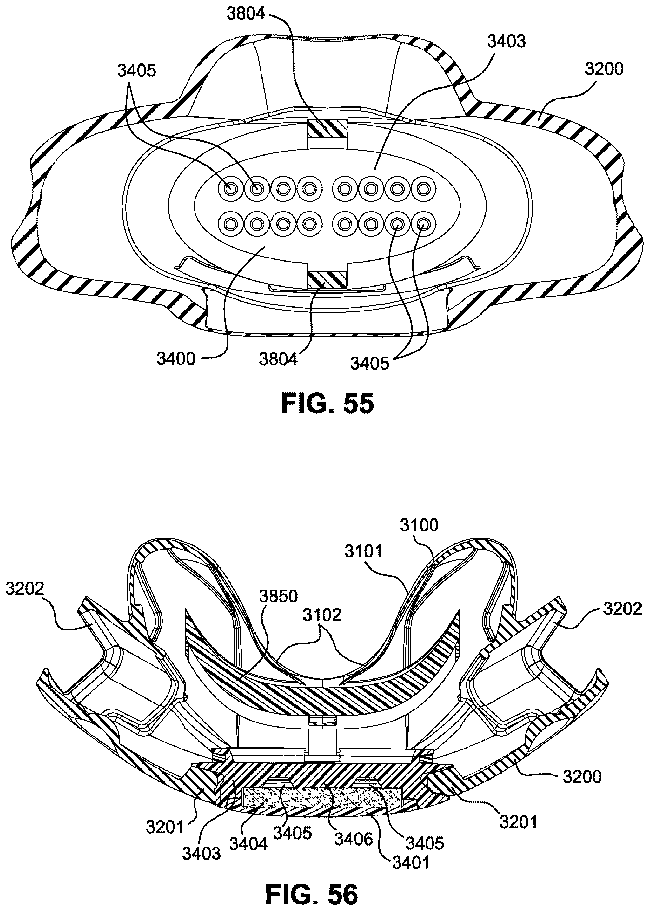

Fig. 55 shows a cross-sectional view taken through line 55-55 of Fig. 52 of a seal-forming structure, a plenum chamber, and a vent of a patient interface according to another example of the present technology.
Fig. 56 shows a cross-sectional view taken through line 56-56 of Fig. 52 of a seal-forming structure, a plenum chamber, and a vent of a patient interface according to another example of the present technology.

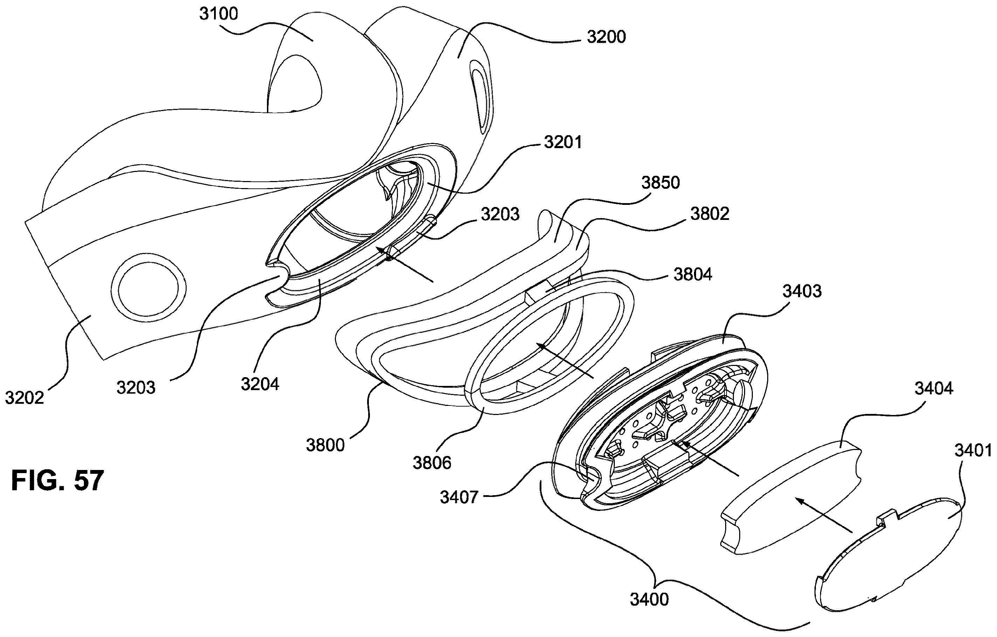

Fig. 57 shows an exploded view of a seal-forming structure, a plenum chamber, and a vent of a patient interface according to another example of the present technology.

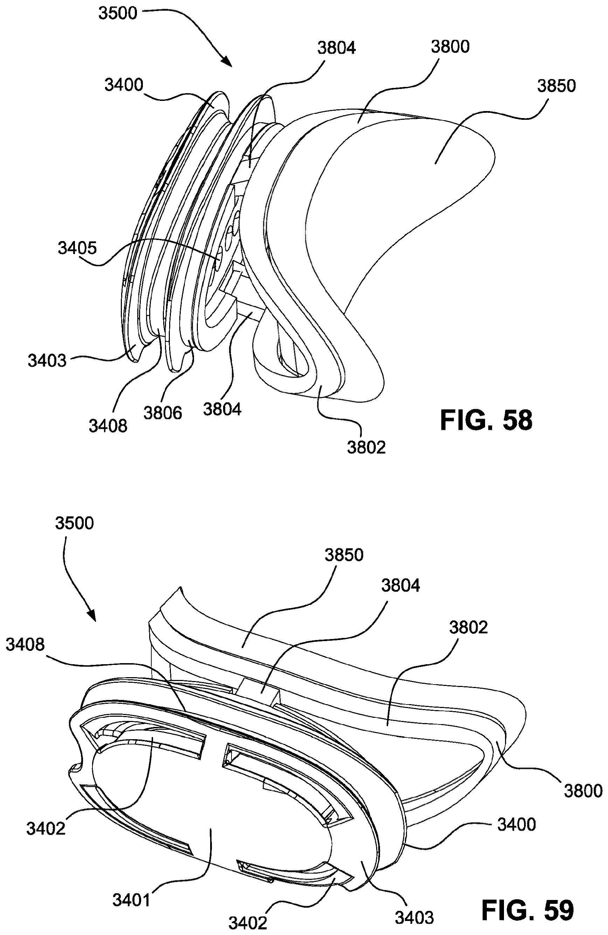

Fig. 58 shows a rear perspective view of an HMX and vent assembly according to an example of the present technology.
Fig. 59 shows a perspective view of an HMX and vent assembly according to an example of the present technology.

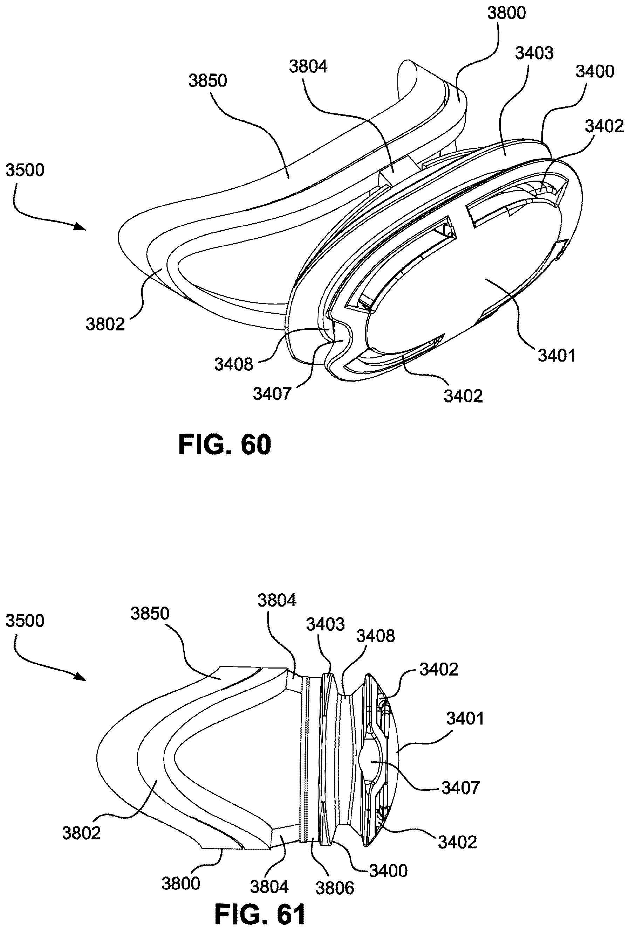

Fig. 60 shows another perspective view of an HMX and vent assembly according to an example of the present technology.
Fig. 61 shows a side view of an HMX and vent assembly according to an example of the present technology.

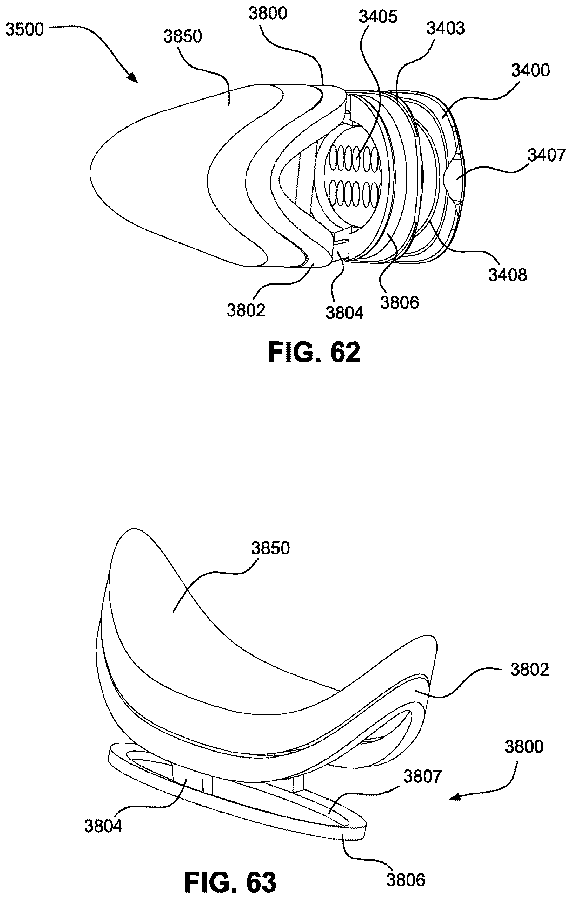

Fig. 62 shows a side perspective view of an HMX and vent assembly according to an example of the present technology.
Fig. 63 shows a rear perspective view of an HMX assembly according to an example of the present technology.

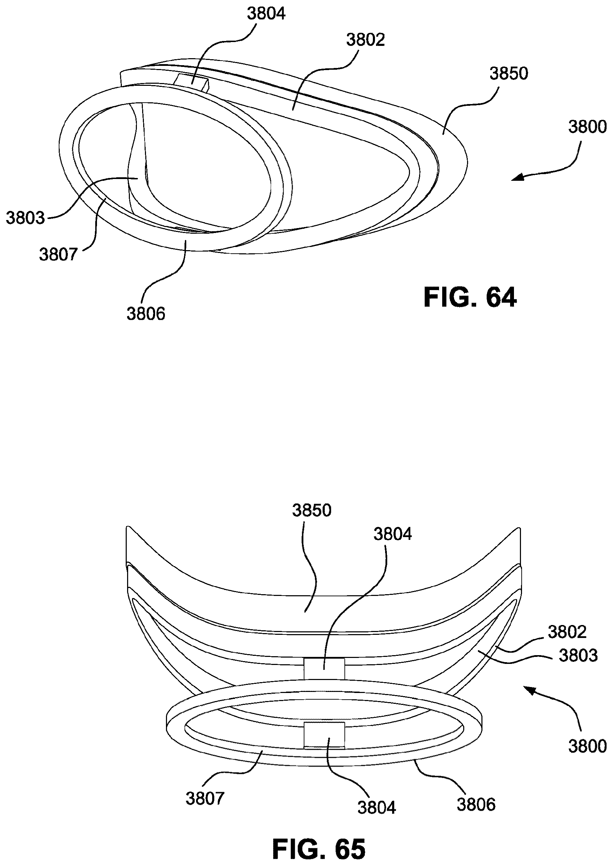

Fig. 64 shows a perspective view of an HMX assembly according to an example of the present technology.
Fig. 65 shows another perspective view of an HMX assembly according to an example of the present technology.

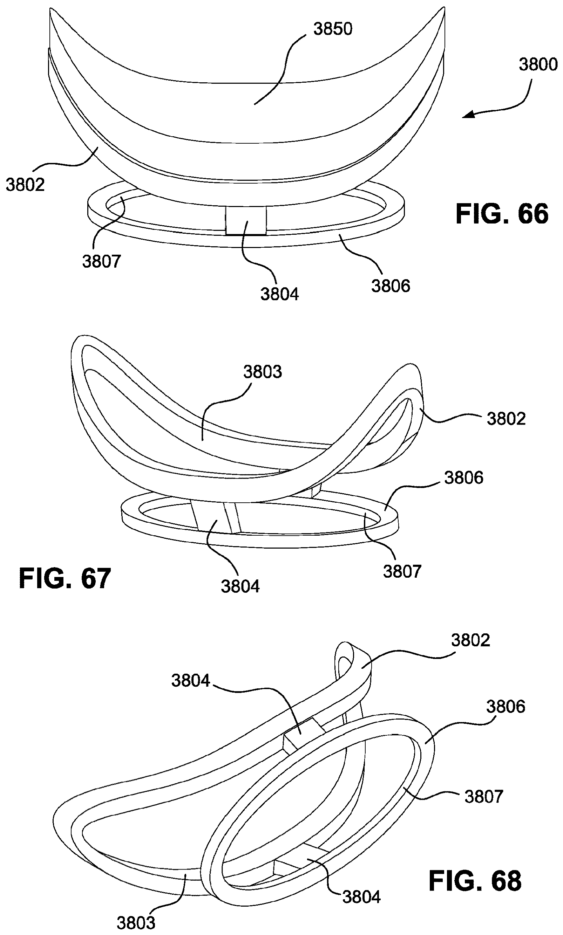

Fig. 66 shows another rear perspective view of an HMX assembly according to an example of the present technology.
Fig. 67 shows a rear perspective view of an HMX frame according to an example of the present technology.
Fig. 68 shows a perspective view of an HMX frame according to an example of the present technology.

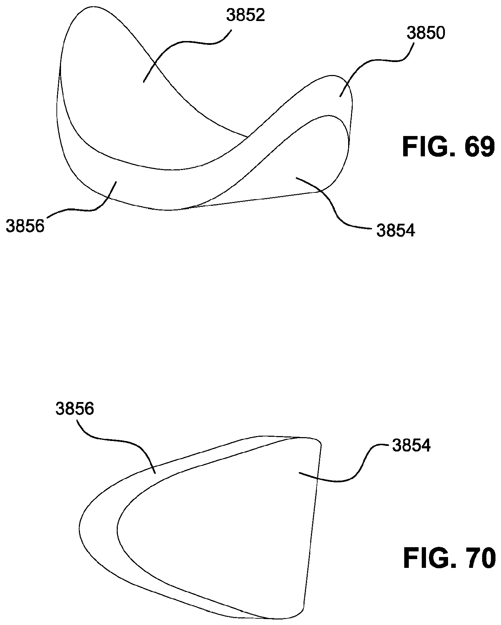

Fig. 69 shows a perspective view of an HMX material according to an example of the present technology.
Fig. 70 shows a side view of an HMX material according to an example of the present technology.

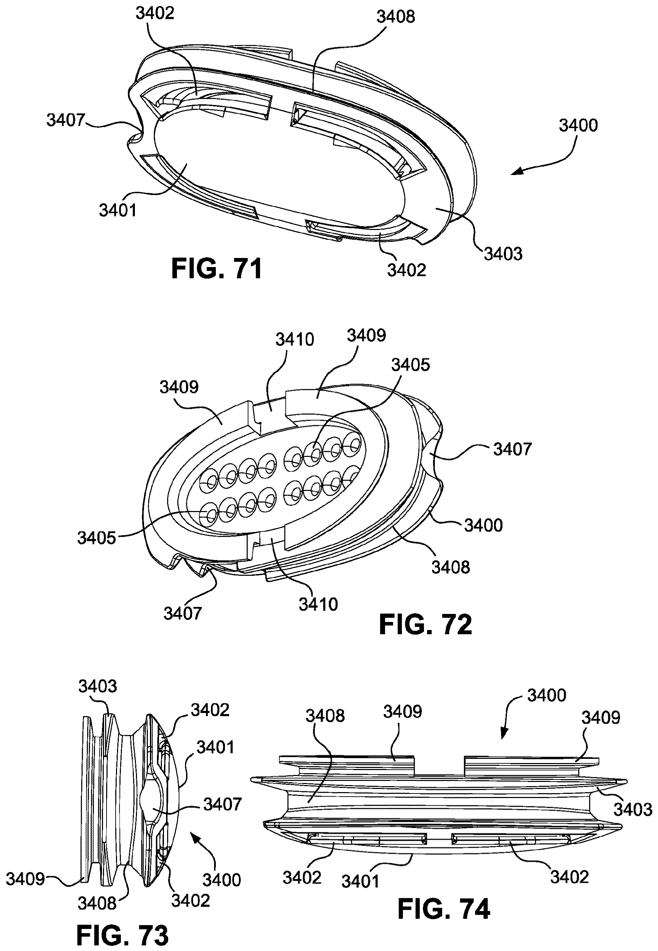

Fig. 71 shows a perspective view of a vent according to an example of the present technology.
Fig. 72 shows a rear perspective view of a vent according to an example of the present technology.
Fig. 73 shows a side view of a vent according to an example of the present technology.
Fig. 74 shows a top view of a vent according to an example of the present technology.

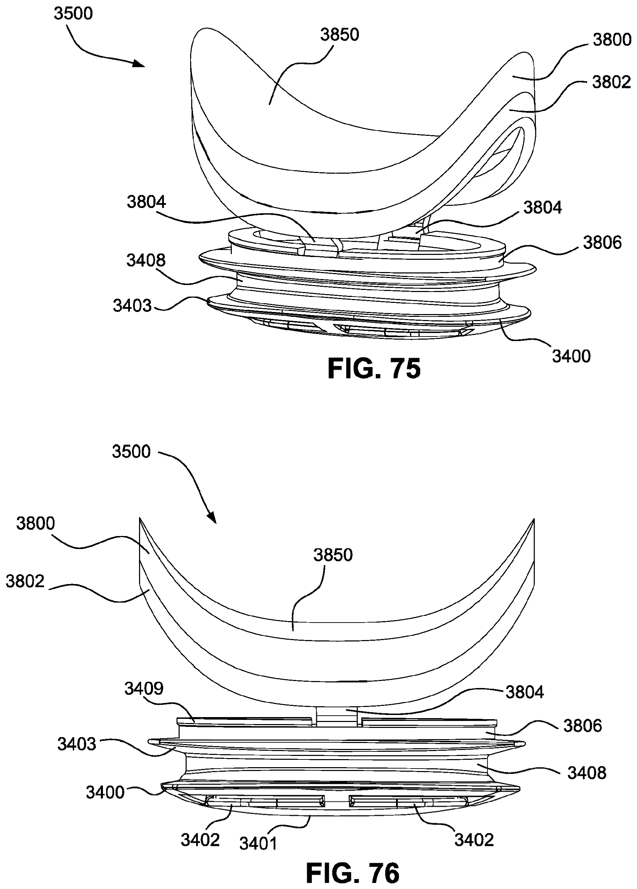

Fig. 75 shows a rear perspective view of an HMX and vent assembly according to another example of the present technology.
Fig. 76 shows a top view of an HMX and vent assembly according to an example of the present technology.

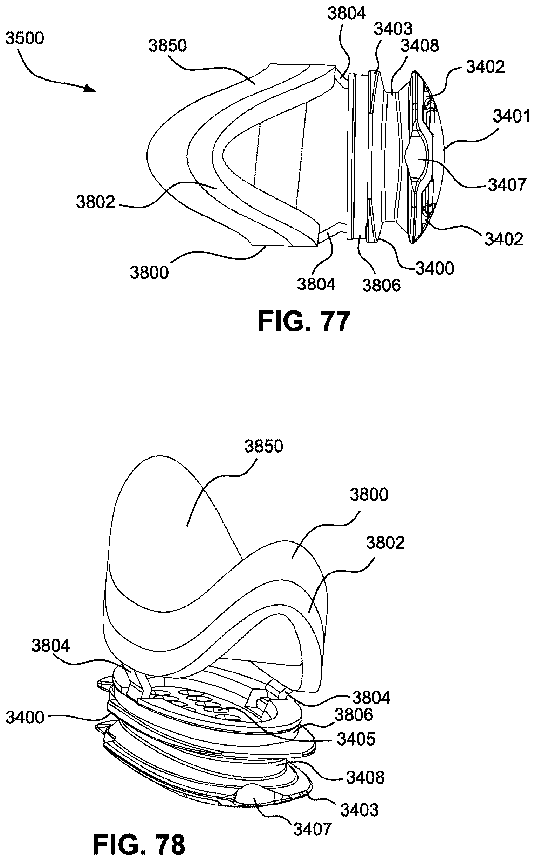

Fig. 77 shows a side view of an HMX and vent assembly according to an example of the present technology.
Fig. 78 shows another rear perspective view of an HMX and vent assembly according to another example of the present technology.

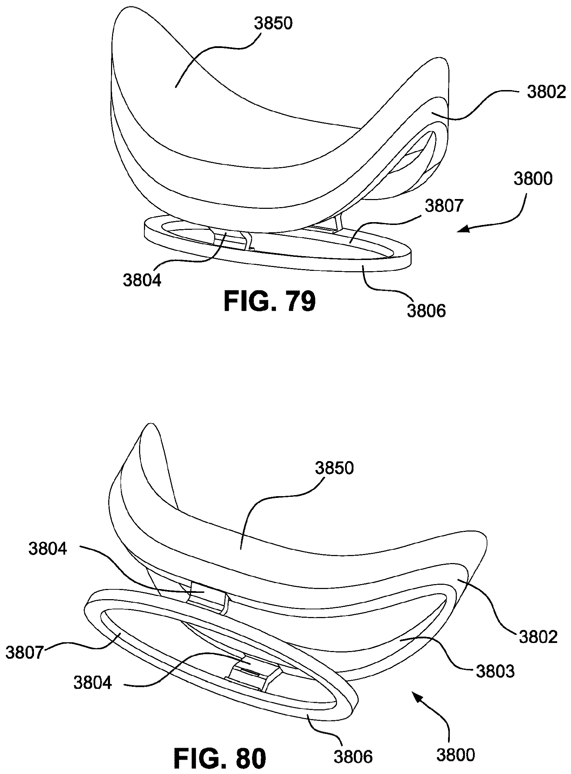

Fig. 79 shows a rear perspective view of an HMX assembly according to an example of the present technology.
Fig. 80 shows a perspective view of an HMX assembly according to an example of the present technology.

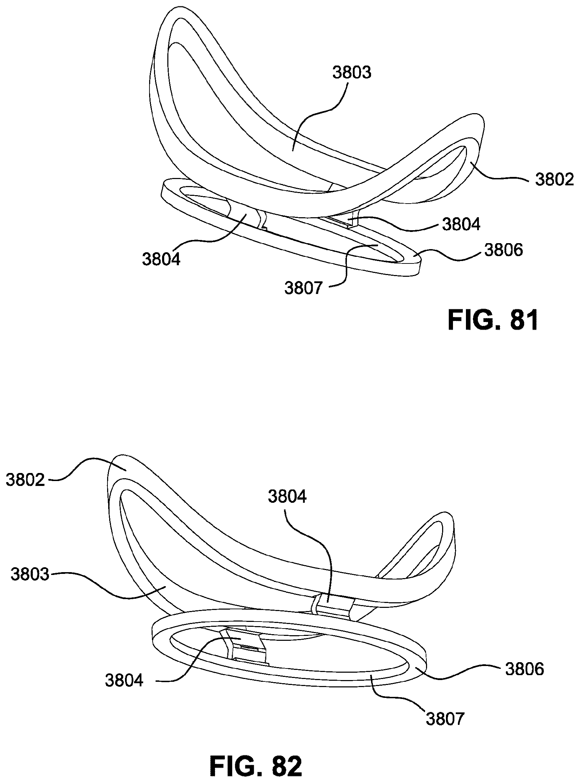

Fig. 81 shows a rear perspective view of an HMX frame according to an example of the present technology.
Fig. 82 shows a perspective view of an HMX frame according to an example of the present technology.

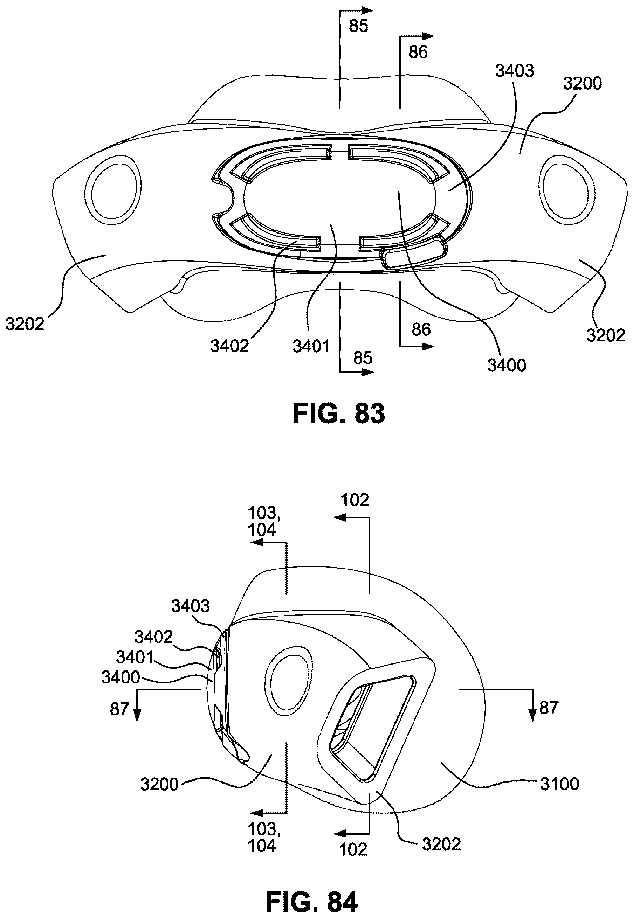

Fig. 83 shows a front view of a seal-forming structure, a plenum chamber, and a vent of a patient interface according to another example of the present technology.
Fig. 84 shows a front view of a seal-forming structure, a plenum chamber, and a vent of a patient interface according to another example of the present technology.

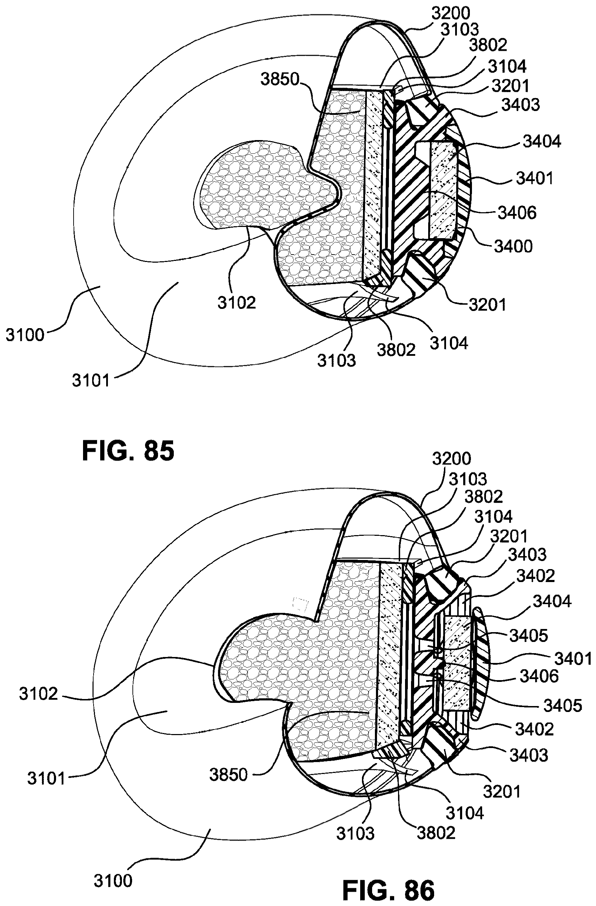

Fig. 85 shows a cross-sectional view taken through line 85-85 of Fig. 84 of a seal-forming structure, a plenum chamber, and a vent of a patient interface according to another example of the present technology.
Fig. 86 shows a cross-sectional view taken through line 86-86 of Fig. 84 of a seal-forming structure, a plenum chamber, and a vent of a patient interface according to another example of the present technology.

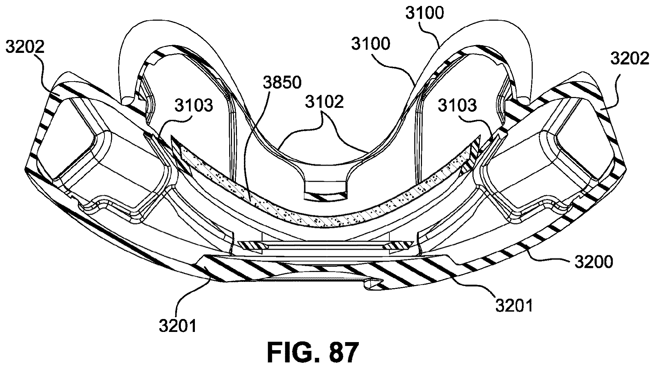

Fig. 87 shows a cross-sectional view taken through line 87-87 of Fig. 84 of a seal-forming structure, a plenum chamber, and a vent of a patient interface according to another example of the present technology.

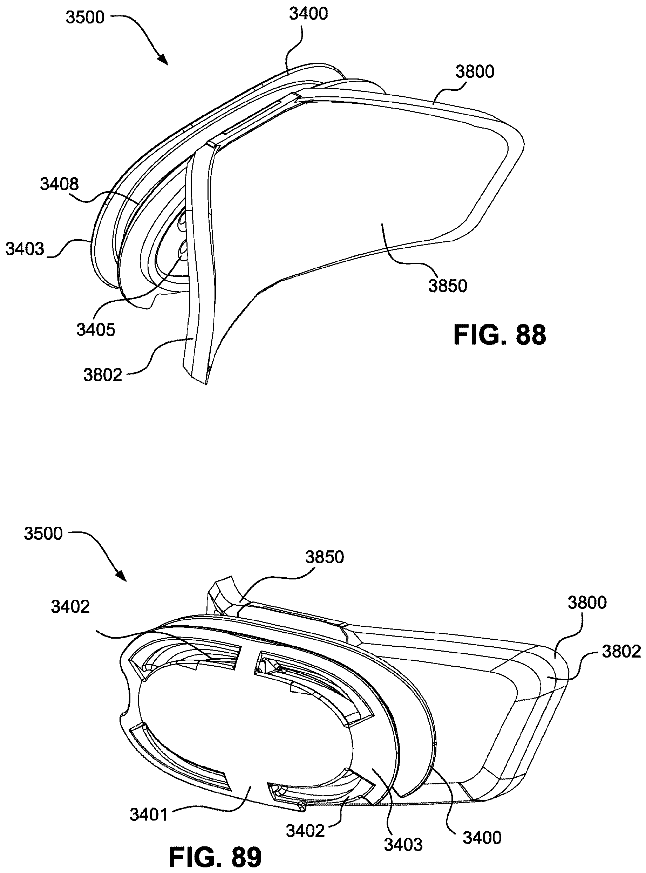

Fig. 88 shows a rear perspective view of an HMX and vent assembly according to an example of the present technology.
Fig. 89 shows a perspective view of an HMX and vent assembly according to an example of the present technology.

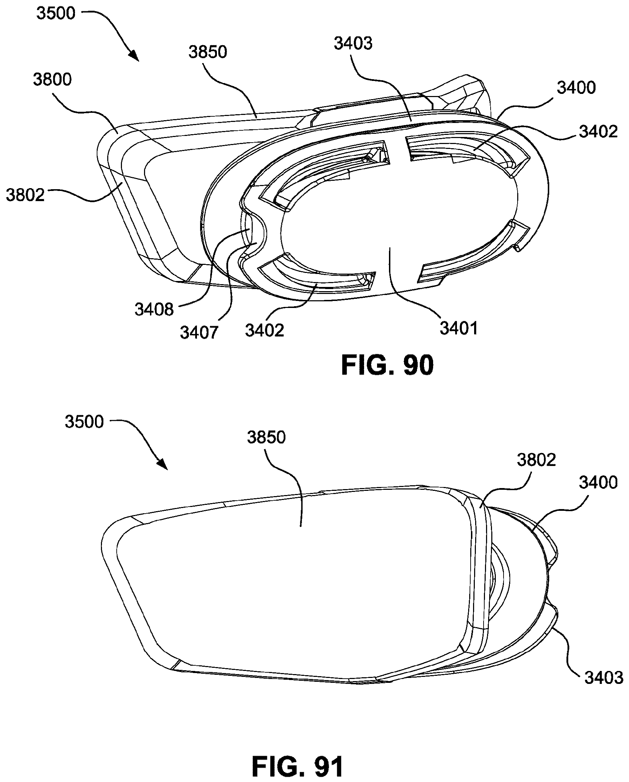

Fig. 90 shows another perspective view of an HMX and vent assembly according to an example of the present technology.
Fig. 91 shows another rear perspective view of an HMX and vent assembly according to an example of the present technology.

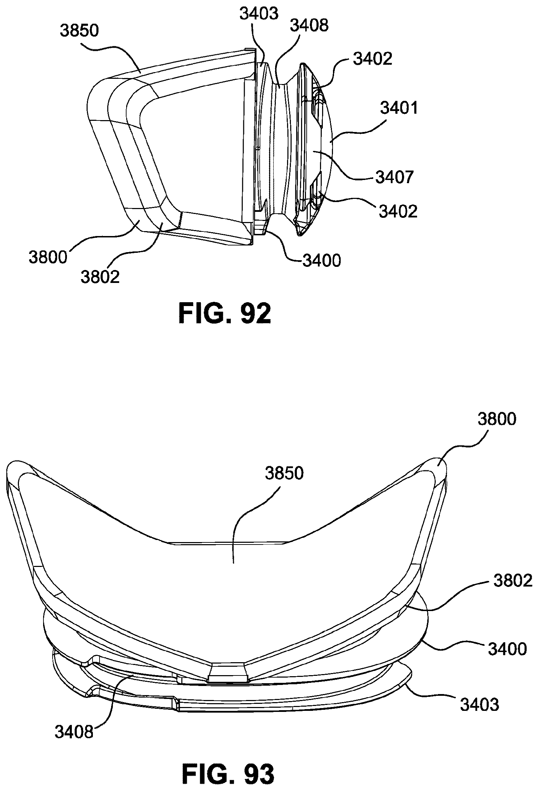

Fig. 92 shows a side view of an HMX and vent assembly according to an example of the present technology.
Fig. 93 shows a rear view of an HMX and vent assembly according to an example of the present technology.

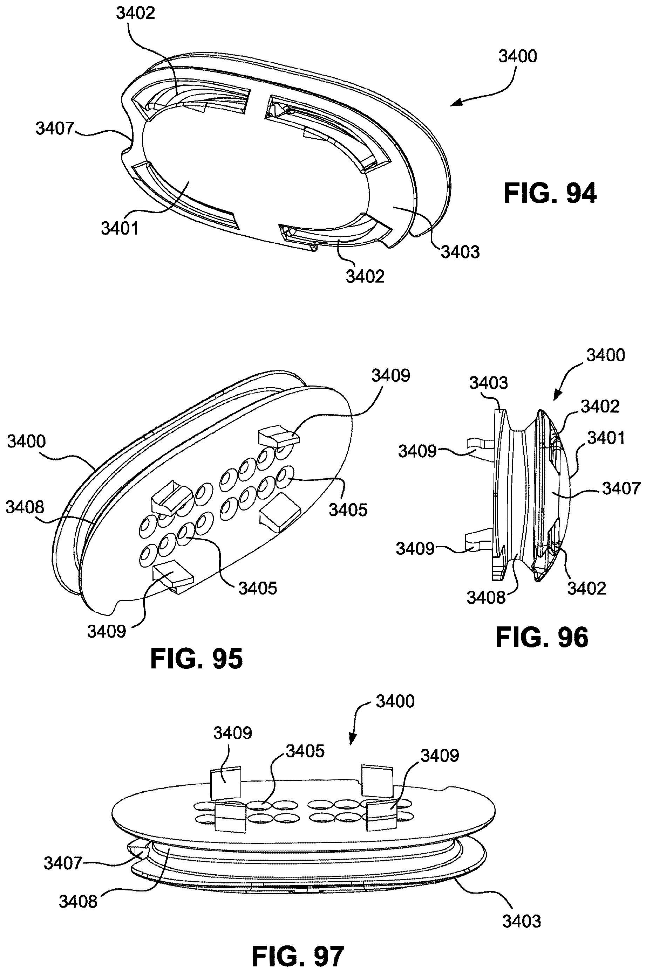

Fig. 94 shows a perspective view of a vent according to an example of the present technology.
Fig. 95 shows a rear perspective view of a vent according to an example of the present technology.
Fig. 96 shows a side view of a vent according to an example of the present technology.
Fig. 97 shows another rear perspective view of a vent according to an example of the present technology.

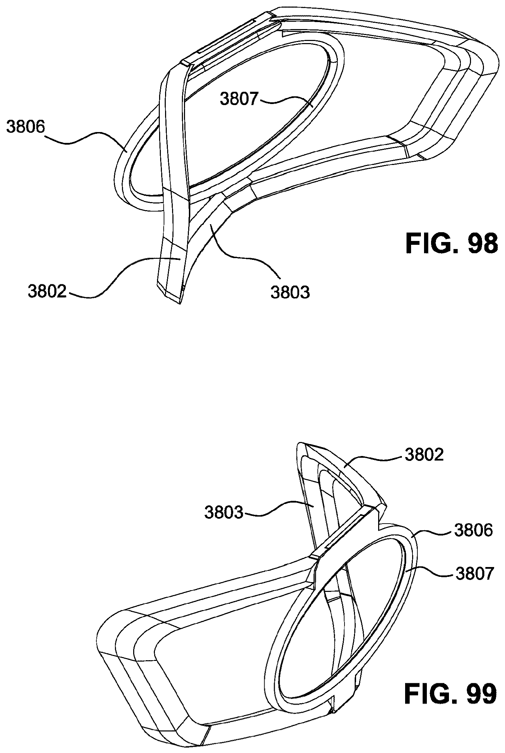

Fig. 98 shows a rear perspective view of an HMX frame according to an example of the present technology.
Fig. 99 shows a perspective view of an HMX frame according to an example of the present technology.

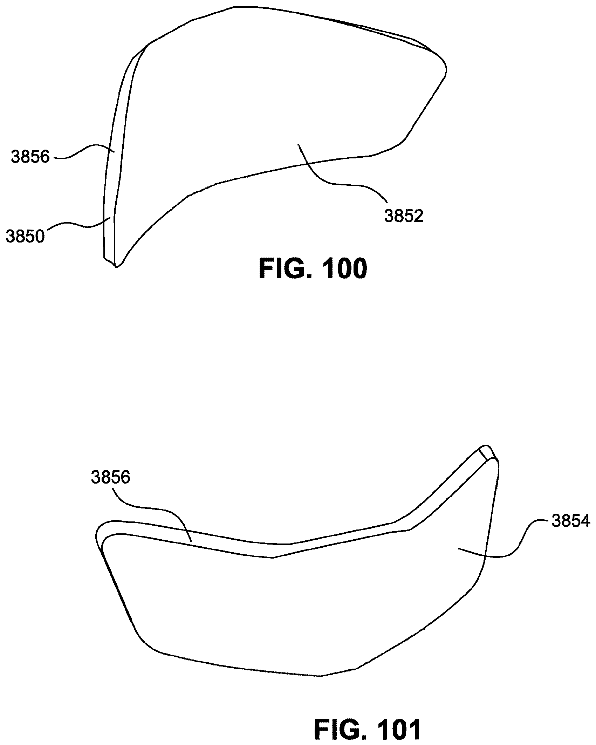

Fig. 100 shows a rear perspective view of an HMX material according to an example of the present technology.
Fig. 101 shows a perspective view of an HMX material according to an example of the present technology.

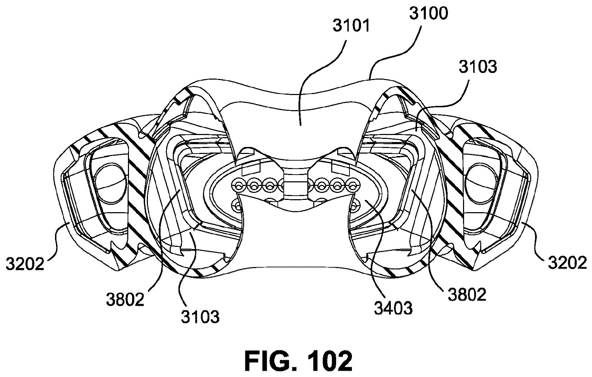

Fig. 102 shows a cross-sectional view taken through line 102-102 of Fig. 84 of a seal-forming structure, a plenum chamber, an HMX assembly, and a vent of a patient interface according to another example of the present technology.

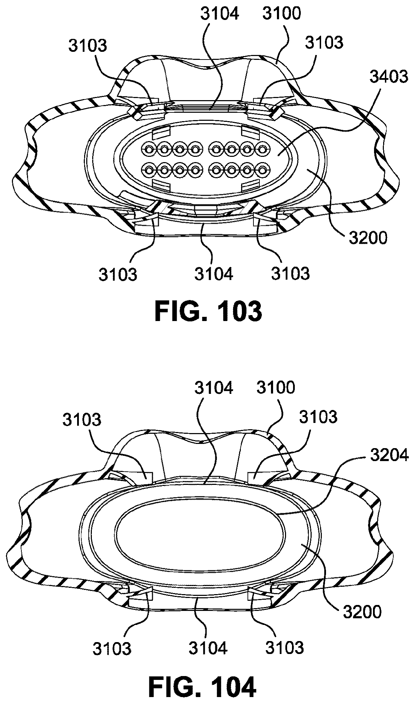

Fig. 103 shows a cross-sectional view taken through line 103, 104 -103, 104 of Fig. 84 of a seal-forming structure, a plenum chamber, and a vent of a patient interface according to another example of the present technology.
Fig. 104 shows a cross-sectional view taken through line 103, 104 -103, 104 of Fig. 84 of a seal-forming structure and a plenum chamber according to another example of the present technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising pressuring air to a positive pressure relative to ambient and directing the pressurized air to the entrance of the airways of a patient 1000.

In certain examples of the present technology, a supply of air at positive pressure relative to ambient is provided to the nasal passages of the patient via one or both nares. In further examples, the supply of air at positive pressure may be provided to the mouth, in addition to the nasal passages.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

A non-invasive patient interface 3000 in accordance with one aspect of the present technology comprises one or more of the following functional aspects: a seal-forming structure 3100, a plenum chamber 3200, a positioning and stabilising structure 3300, a vent assembly 3400, one form of connection port 3600 for connection to air circuit 4170, and a forehead support 3700. In some forms, a functional aspect may be performed by one or more physical components. In some forms, one physical component may perform one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 4 cmH₂O with respect to ambient.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

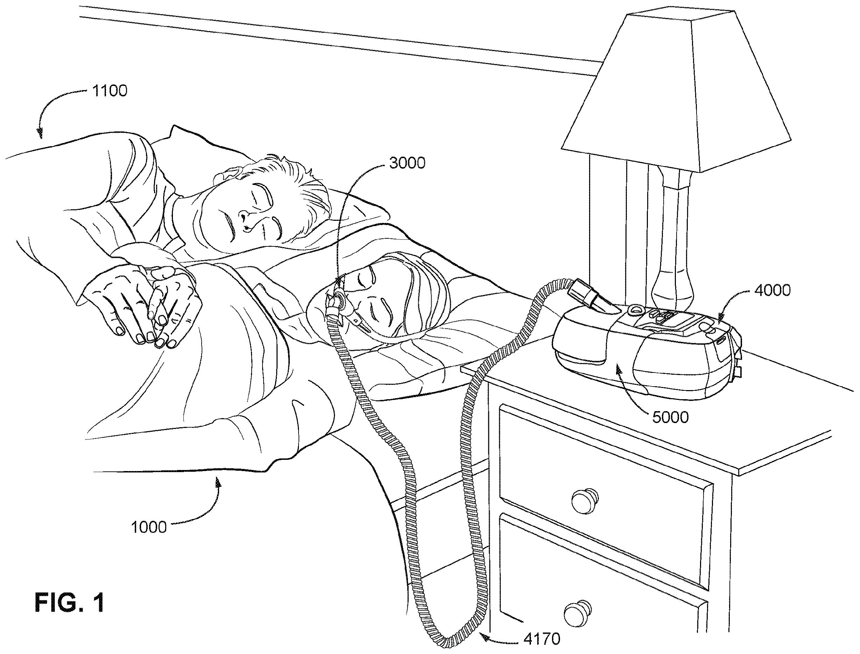

As shown in Figs. 43-47, a non-invasive patient interface 3000 in accordance with one aspect of the present technology comprises the following functional aspects: a seal-forming structure 3100, a plenum chamber 3200, a positioning and stabilising structure 3300, a vent assembly 3400 and one form of connection port 3600 for connection to an air circuit (e.g. the air circuit 4170 shown in Figs. 1A-1C). In this example, the seal-forming structure 3100 and the plenum chamber 3200 are provided by a cushion module 3150.

### 5.3.1 Seal-forming structure

In one form of the present technology, a seal-forming structure 3100 includes a target seal-forming region, and may additionally include a cushioning function. The target seal-forming region is a region on the seal-forming structure 3100 where sealing may occur. The region where sealing actually occurs - the actual sealing surface - may change within a given treatment session, from day to day, and from patient to patient, depending on a range of factors including for example, where the patient interface 3000 was placed on the face, tension in the positioning and stabilising structure 3300, and the shape of a patient's face.

In one form, the target seal-forming region is located on an outside surface of the seal-forming structure 3100.

In certain forms of the present technology, the seal-forming structure 3100 is constructed from a biocompatible material, e.g., silicone rubber.

A seal-forming structure 3100 in accordance with the present technology may be constructed from a soft, flexible, resilient material such as silicone.

In certain forms of the present technology, a system comprising more than one seal-forming structure 3100, each being configured to correspond to a different size and/or shape range. For example, the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure 3100 includes a sealing flange utilizing a pressure-assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that seals in use on a nose bridge region or on a nose-ridge region of the patient's face.

In one form, the seal-forming structure includes a saddle-shaped region constructed to seal in use on a nose bridge region or on a nose-ridge region of the patient's face.

### 5.3.1.3 Upper lip region

In one form, the non-invasive patient interface 3000 comprises a seal-forming structure that seals in use on an upper lip region (that is, the lip superior) of the patient's face.

In one form, the seal-forming structure includes a saddle-shaped region constructed to seal in use on an upper lip region of the patient's face.

### 5.3.1.4 Chin-region

In one form the non-invasive patient interface 3000 comprises a seal-forming structure that seals in use on a chin-region of the patient's face.

In one form, the seal-forming structure includes a saddle-shaped region constructed to seal in use on a chin-region of the patient's face.

### 5.3.1.5 Forehead region

In one form, the seal-forming structure that seals in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to seal with a respective naris of the nose of a patient.

Nasal pillows in accordance with an aspect of the present technology include: a frusto-cone, at least a portion of which seals on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

In one form the seal-forming structure 3100 of the non-invasive patient interface 3000 comprises a pair of nasal puffs, or nasal pillows, each nasal puff or nasal pillow being constructed and arranged to form a seal with a respective naris of the nose of a patient. Figs. 46 and 47 show a patient interface 3000 having a seal-forming structure 3100 provided by a pillows cushion module 3160. The pillows cushion module 3160 comprises a pair of nasal pillows 3165. In this example, the same positioning structure 3300 as shown in Figs. 43-45 is used to hold the pillows cushion module 3160 in sealing contact with the patient's nose. The same concepts and features of the positioning and stabilising structure 3300 described with reference to the cradle cushion module 3150 may be applied to a positioning and stabilising structure 3300 configured to be used with the pillows cushion module 3160 (or another type of cushion module such as a full face cushion module, oro-nasal cushion module, ultra-compact full face cushion module, nasal cushion module and the like).

Nasal pillows 3165 in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.1.7 Nasal Cradle

In one form, for example as shown in Figs. 43-45, the seal-forming structure 3100 is configured to form a seal in use with the underside of the nose around the nares and optionally with the lip superior of the patient 1000. This type of seal-forming structure may be referred to as a "cradle cushion" or "sub-nasal mask". The shape of the seal-forming structure may be configured to match or closely follow the underside of the patient's nose, i.e. the profile and angle of the seal-forming structure may be substantially parallel to the patient's naso-labial angle. In one form of nasal cradle cushion, the seal-forming structure comprises a bridge portion defining two orifices, each of which, in use, supplies air or breathable gas to a different one of the patient's nares. The bridge portion may be configured to contact or seal against the patient's columella in use. In some forms of the technology, the seal-forming structure 3100 is configured to form a seal on an underside of the patient's nose without contacting a nasal bridge region of the patient's nose. In some examples, patient interface may comprise a seal-forming structure 3100 in the form of a cradle cushion as described in U.S. Publication No. US 2020/0054850 A1, the entire contents of which are incorporated herein by reference.

### 5.3.1.8 Nasal Mask Cushion

In one form, the non-invasive patient interface 3000 comprises a seal-forming portion that forms a seal in use on an upper lip region (that is, the *lip superior*), a nasal bridge region and a cheek region of the patient's face. This is the case, for example, with the patient interface 3000 shown in Fig. 1B. This seal-forming portion delivers a supply of air or breathable gas to both nares of patient 1000 through a single orifice. This type of seal-forming structure may be referred to as a "nasal cushion" or "nasal mask". In some examples of the present technology, the positioning and stabilising structure 3300 shown in Figs. 43-47 may be utilised to hold a nasal cushion in sealing position on a patient's face.

### 5.3.1.9 Full-face Mask Cushion

In one form the patient interface 3000 comprises a seal-forming portion that forms a seal in use on a chin-region, a nasal bridge region and a cheek region of the patient's face. This is the case, for example, with the patient interface 3000 shown in Fig. 1C. This seal-forming portion delivers a supply of air or breathable gas to both nares and mouth of patient 1000 through a single orifice. This type of seal-forming structure may be referred to as a "full-face mask". In some examples of the present technology, the positioning and stabilising structure 3300 shown in Figs. 43-47 may be utilised to hold a full-face cushion in sealing position on a patient's face.

### 5.3.1.10 Oronasal Mask Cushion

In another form the patient interface 3000 comprises a nasal seal-forming structure in the manner of a nasal cushion or nasal cradle cushion and an oral seal-forming structure that is configured to form a seal in use around the mouth of a patient (which may be referred to as a "mouth cushion" or "oral mask"). In such a mask air or breathable gas is supplied in use through separate orifices to the patient's nares and the patient's mouth. This type of seal-forming structure 3100 may be referred to as an "oronasal cushion" or "ultra-compact full face cushion". In one form, the nasal seal-forming structure and oral seal-forming structure are integrally formed as a single component. In some examples, patient interface may comprise a seal-forming structure 3100 in the form of a cradle cushion as described in International Publication No. WO 2019/183680 A1, the entire contents of which are incorporated herein by reference.

### 5.3.2 Plenum chamber

The plenum chamber 3200 may have a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and/or more comfortable for the wearer, which can improve compliance with therapy.

In certain forms of the present technology, the plenum chamber 3200 is constructed from a transparent material, e.g., polycarbonate. The use of a transparent material can reduce the obtrusive appearance of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusive appearance of the patient interface, and help improve compliance with therapy.

The plenum chamber 3200 in examples of the present technology may be constructed from a relatively flexible material such as silicone rubber. In further examples, the seal-forming structure 3100 and the plenum chamber may be constructed from a single piece of homogenous material, such as relatively flexible material, which may be silicone rubber. In further examples, the seal-forming structure 3100 and the plenum chamber 3200 may be formed in one piece but the material of each may have a different mechanical characteristic, e.g., both may be made from silicone rubber with the seal-forming structure 3100 having a lower durometer than the plenum chamber 3200. The plenum chamber 3200 may also include tube connectors 3202 to connect to corresponding tubes, as shown in Figs. 43-47, to receive pressurised air.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 retains the patient interface 3000 on the patient's head with a force at least sufficient to overcome the effect of the positive pressure in the plenum chamber 3200 urging the seal-forming structure 3100 to lift off the face.

In one form the positioning and stabilising structure 3300 retains the patient interface 3000 on the patient's head with a force sufficient to overcome the effect of the gravitational force on the patient interface 3000.

In one form the positioning and stabilising structure 3300 retains the patient interface 3000 on the patient's head with a force as a safety margin to overcome the potential effect of disrupting forces on the patient interface 3000, such as from tube drag, or accidental interference with the patient interface.

In one form of the present technology, a positioning and stabilising structure 3300 is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is configured so as not to be too large and bulky to prevent the patient from lying in a supine sleeping position with a back region of the patient's head on a pillow.

In one form of the present technology, a positioning and stabilising structure 3300 is configured so as not to be too large and bulky to prevent the patient from lying in a side sleeping position with a side region of the patient's head on a pillow.

In one form of the present technology, a positioning and stabilising structure 3300 includes a decoupling portion located between an anterior portion of the positioning and stabilising structure 3300, and a posterior portion of the positioning and stabilising structure 3300. The decoupling portion does not resist compression and may be a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone. The first tie may be provided, for example, as part of a patient interface that comprises a cradle cushion, nasal pillows, nasal cushion, full-face cushion or an oronasal cushion. For example, as shown in Figs. 43-47, the positioning and stabilising structure 3300 comprises a first tie in the form of tubes 3350 which lie over the top of the patient's head.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and non-rigid. An advantage of this aspect is that the strap is more comfortable for a patient to lie upon while the patient is sleeping. As shown in Figs. 43-47, the positioning and stabilising structure 3300 comprises a strap 3310 that is bendable. The strap 3310 may be considered a backstrap. The strap 3310 is sufficiently flexible to pass around the back of the patient's head and lie comfortably against the patient's head, even when under tension in use.

In certain forms of the present technology, a positioning and stabilising structure 3300 comprises a strap constructed to be breathable to allow water vapour to be transmitted through the strap.

In certain forms of the present technology, a system is provided comprising more than one positioning and stabilizing structure 3300, each being configured to provide a retaining force to correspond to a different size and/or shape range. For example, the system may comprise one form of positioning and stabilizing structure 3300 suitable for a large sized head, but not a small sized head, and another. suitable for a small sized head, but not a large sized head.

### 5.3.3.1 Headgear tubing

In some forms of the present technology, the positioning and stabilising structure 3300 comprises one or more tubes 3350 that deliver pressurised air received from a conduit forming part of the air circuit 4170 from the RPT device to the patient's airways, for example through the plenum chamber 3200 and seal-forming structure 3100. In the form of the present technology illustrated in Figs. 43-47, the positioning and stabilising structure 3300 comprises two tubes 3350 that deliver air to the seal-forming structure 3100 from the air circuit 4170. The tubes 3350 are an integral part of the positioning and stabilising structure 3300 of patient interface 3000 to position and stabilise the seal-forming structure 3100 of the patient interface to the appropriate part of the patient's face (for example, the nose and/or mouth). This allows the conduit of air circuit 4170 providing the flow of pressurised air to connect to a connection port 3600 of the patient interface in a position other than in front of the patient's face which may be unsightly to some people. While a pair of tubes 3350 have some advantages (described below), in some examples, the positioning and stabilising structure 3300 comprises only a single tube 3350 configured to overlie the patient's head on one side. A strap or other stabilising component may be provided to the other side of the patient's head between the top end of the single tube 3350 and the seal-forming structure 3100, to provide balanced forces on the seal-forming structure 3100.

Since air can be contained and passed through headgear tubing 3350 in order to deliver pressurised air from the air circuit 4170 to the patient's airways, the positioning and stabilising structure 3300 may be described as being inflatable. It will be understood that an inflatable positioning and stabilising structure 3300 does not require all components of the positioning and stabilising structure 3300 to be inflatable. For example, in the example shown in Figs. 43-47, the positioning and stabilising structure 3300 comprises the headgear tubing 3350, which is inflatable, and the strap 3310, which is not inflatable.

In certain forms of the present technology, the patient interface 3000 may comprise a connection port 3600 located proximal a top, side or rear portion of a patient's head. For example, in the form of the present technology illustrated in Figs. 43-47, the connection port 3600 is located on top of the patient's head. In this example the patient interface 3000 comprises an elbow 3610 to which the connection port 3600 is provided. The elbow 3610 may swivel with respect to the positioning and stabilising structure 3300 and order to decouple movement of a conduit connected to the connection port 3600 from the positioning and stabilising structure 3300. Additionally, or alternatively, a conduit connected to the connection port 3600 may swivel with respect to the elbow 3610. In the illustrated example, elbow 3610 comprises a swivelling conduit connector to which a conduit of the air circuit 4170 is able to connect such that the conduit can rotate about its longitudinal axis with respect to the elbow 3610.

Patient interfaces in which the connection port is not positioned in front of the patient's face may be advantageous as some patients find a conduit that connects to a patient interface in front of the face to be unsightly and obtrusive. For example, a conduit connecting to a patient interface in front of the face may be prone to being tangled up in bedclothes or bed linen, particularly if the conduit extends downwardly from the patient interface in use. Forms of the technology with a patient interface with a connection port positioned proximate the top of the patient's head in use may make it easier or more comfortable for a patient to lie or sleep in one or more of the following positions: in a side or lateral position; in a supine position (i.e. on their back, facing generally upwards); and in a prone position (i.e. on their front, facing generally downwards). Moreover, connecting a conduit to the front of a patient interface may exacerbate a problem known as tube drag, wherein the conduit may provide an undesired drag force upon the patient interface thereby causing dislodgement away from the face.

In the form of the present technology illustrated in Figs. 43-47, the positioning and stabilising structure 3300 comprises two tubes 3350, each tube 3350 being positioned in use on a different side of the patient's head and extending across the respective cheek region, above the respective ear (superior to the otobasion superior on the patient's head) to the elbow 3610 on top of the head of the patient 1000. This form of technology may be advantageous because, if a patient sleeps with their head on its side and one of the tubes is compressed to block or partially block the flow of gas along the tube, the other tube remains open to supply pressurised gas to the patient. In other examples of the technology, the patient interface 3000 may comprise a different number of tubes, for example one tube, or three or more tubes. In one example in which the patient interface has one tube 3350, the single tube 3350 is positioned on one side of the patient's head in use (e.g. across one cheek region) and a strap forms part of the positioning and stabilising structure 3300 and is positioned on the other side of the patient's head in use (e.g. across the other region) to assist in securing the patient interface 3000 on the patient's head.

In the form of the technology shown in Figs. 43-47, the two tubes 3350 are fluidly connected at their upper ends to each other and to connection port 3600. In one embodiment, the two tubes are integrally formed while in other embodiments the tubes are separate components that are connected together in use and may be disconnected, for example for cleaning or storage. Where separate tubes are used they may be indirectly connected together, for example each may be connected to a T-shaped conduit having two conduit arms each fluidly connectable to the tubes 3350 and a third conduit arm or opening acting as the connection port 3600 and connectable in use to the air circuit 4170. The connection port 3600 may comprise an elbow 3610 received in fluid connection opening 3390 at the centre of two integrally formed tubes 3350. The elbow 3610 may be received in a ring in the fluid connection opening 3390 and may be configured to swivel within the ring. The fluid connection opening 3390 may be also considered a connection port 3600 itself.

The tubes 3350 may be formed of a semi-rigid material such as an elastomeric material, e.g. silicone. The tubes may have a natural, preformed shape and be able to be bent or moved into another shape if a force is applied to the tubes. For example, the tubes may be generally arcuate or curved in a shape approximating the contours of a patient's head between the top of the head and the nasal or oral region.

The positioning and stabilising structure 3300 in some examples may comprise sleeves 3364 around the tubes 3350. For example, as shown in 43-47, sleeves 3364 are provided to the non-extendable tube sections 3363. In some examples, the patient interface 3000 may not comprise sleeves 3364 and in other examples the patient interface 3000 may comprise sleeves 3364 that cover more, or all, of the tubes 3350. The sleeves 3364 may be formed to fit to the curved shape of the tubes 3350. In some examples, the sleeves 3364 are formed from a smooth fabric. The sleeves 3364 may be more comfortable against the patient's face than the tube 3350 without any covering.

As described in US Patent No. 6,044,844, the contents of which are incorporated herein, the tubes 3350 may be crush resistant to avoid the flow of breathable gas through the tubes if either is crushed during use, for example if it is squashed between a patient's face and pillow. Crush resistant tubes may not be necessary in all cases as the pressurised gas in the tubes may act as a splint to prevent or at least restrict crushing of the tubes 3350 during use. A crush resistant tube may be advantageous where only a single tube 3350 is present as if the single tube becomes blocked during use the flow of gas would be restricted and therapy will stop or reduce in efficacy.

In certain forms of the technology, one or more portions of the tubes 3350 may be rigidised by one or more rigidising or stiffening elements. Examples of rigidising elements include: sections of the tubes 3350 that are comparatively thicker than other sections; sections of the tubes 3350 that are formed from a material that is comparatively more rigid that the material forming other sections; and a rigid member attached to the inside, outside or embedded in a section of tube. The use of such rigidising elements helps to control how the positioning and stabilising structure 3300 will function in use, for example where the tubes 3350 is more likely to deform if forces are applied to them and where the shape of the tubes 3350 is more likely to be maintained if forces are applied. The selection of where such rigidising elements are positioned in the tubes 3350 can therefore help to promote comfort when the patient interface 3000 is worn and can help to maintain a good seal at the seal-forming structure 3100 during use. Rigidising or stiffening elements may be in positioning and stabilising structures 3300 which are configured to support relatively heavy seal-forming structures such as full face or oro-nasal cushion assemblies.

The tubes 3350 in the form of the technology shown in Figs. 43-47 have a length of between 15 and 30cm each, for example between 20 and 27cm each. In one example each of the tubes are around 26cm long. In another example each of the tubes is around 23cm long. The length of the tubes is selected to be appropriate for the dimensions of the heads of typical patients, for example the distance between the region proximate the top of the head where the upper end of the tubes 3350 are situated, and the region proximate the openings to the patient's airways at which the lower end of the tubes 3350 connect to the cradle cushion module 3150 (or pillows cushion module 3160) when following a generally arcuate path down the sides of the heads and across the patient's cheek region such as is shown in Figs. 43-47. As described in more detail below, the patient interface 3000 is configured so that the length of the tubes 3350 can be varied in some forms of the technology and the above lengths may apply to the tube in a contracted, stretched or neutral state. It will be appreciated that the length of the tubes 3350 will depend on the length of other components in the patient interface 3000, for example the length of arms of a T-shaped conduit to which the upper ends of tubes 3350 connect and/or the size of the plenum chamber 3200.

### 5.3.3.1.1 Positioning of Headgear Components

Each tube 3350 may be configured to receive a flow of air from the connection port 3600 on top of the patient's head and to deliver the flow of air to the seal-forming structure at the entrance of the patient's airways. In the example of Figs. 43-47, the at least one tube 3350 extends between the seal-forming structure 3100 and the connection port 3600 across the patient's cheek region and above the patient's ear, i.e. a portion of tube 3350 that connects to the cushion module overlays a maxilla region of the patient's head in use and a portion of tube 3350 overlays a region of the patient's head superior to the otobasion superior on the patient's head. Each of the one or more tubes 3350 may also lie over the patient's sphenoid bone and/or temporal bone and either or both of the patient's frontal bone and parietal bone. The connection port 3600 and elbow 3610 may be located in use over the patient's parietal bone, frontal bone or the junction therebetween.

The exemplary form of the technology illustrated in Figs. 43-47 has tubes 3350 which curve around the upper part of the patient's head from the upper end of the tubes 3350 that connect to elbow 3610 on top of the head to the point at which the strap 3310 connects to the tubes 3350 with relatively little curvature in the sagittal plane. In between the point at which the rear headgear strap 3310 connects to the tubes 3350 and the lower ends of the tubes 3350 at which they connect with the cradle cushion module 3150 in front of the patient's airways under the nose, the tubes 3350 curve forwards between the patient's ears and eyes and across the cheek region.

The degree to which the patient interface 3000 fits an individual patient can be altered by varying the length of the tubes 3350 and, alternatively or additionally, by altering the position of the patient interface 3000 or portions thereof on the patient's head. For example, a patient interface 3000 having tubes 3350 of a certain length can be adjusted to better fit a patient by moving portions of the positioning and stabilising structure 3300 in the posterior or anterior direction on the patient's head. For example, positioning the junction of the tubes 3350 above the patient's head further forward (i.e. in the anterior direction) enables a patient interface 3000 having tubes 3350 of a certain length to fit a larger head than if the junction of the tubes 3350 is positioned further backward (i.e. in the posterior direction). In most patient, if the junction of the tubes 3350 is positioned forwardly, the superior portions of the tubes 3350 lie over a smaller portion of the patient's head than if the junction of the tube 3350 is positioned rearwardly.

In certain forms of the present technology the patient interface 3000 is configured such that the connection port 3600 can be positioned in a range of positions across the top of the patient's head so that the patient interface 3000 can be positioned as appropriate for the comfort or fit of an individual patient. One way this can be achieved so that the seal-forming structure 3100 forms an effective seal with the patient's face irrespective of the position of the connection port 3600 on the patient's head is to de-couple movement of the upper portion of the patient interface 3000 from the lower portion of the patient interface 3000. Such de-coupling can be achieved using, for example, mechanisms that allow parts of the headgear tubes 3350 to easily move or flex relative to other parts of the patient interface 3000. Such mechanisms will be described below.

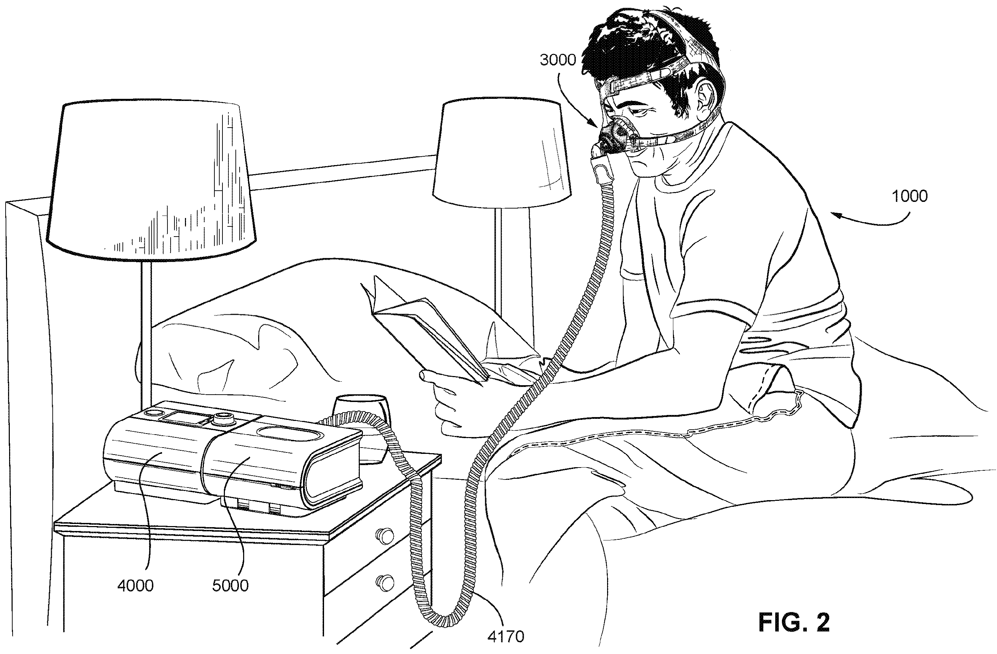

In a certain form of the present technology, the patient interface 3000 is configured such that the connection port 3600 is positioned approximately at a top point of the patient's head. The connection port 3600 may be positioned in the sagittal plane and aligned with the otobasion superior points in a plane parallel to the coronal plane. The otobasion superior points are identified in Fig. 2D. As will be described below, in some forms of the technology, the positioning and stabilising structure 3300 is configured to be worn in different positions, with the effect that the connection port 3600 may be positioned proximate the top of the patient's head in the sagittal plane up to around 20mm forward or 20mm rearward of the otobasion superior points.

In some examples of the present technology, the connection port 3600 may be positioned in the sagittal plane and aligned with a junction between the frontal bone and the parietal bones. The connection port 3600 may be positioned approximately over the junction of the coronal suture and the sagittal suture. In this configuration, the superior portions of the tubes 3350 may lie over and/or along a portion of the coronal suture. However, as mentioned above the patient has the ability to move the connection port 3600 anteriorly or posteriorly in order to adjust the fit of the patient interface 3000.

An advantage provided by the tubes 3350 overlying the patient's head slightly anterior to the superior-most point (e.g. at or proximate the coronal suture) is that the risk of the tubes 3350 riding in a posterior direction in use may be reduced. In many patients there may be a recess or "divot" where the coronal suture meets the sagittal suture. The positioning and stabilising structure 3300 may be particularly stable when tubes 3350 lie within this divot. Accordingly, in some examples the tubes 3350 are configured with appropriate curvature and/or ability to curve in order to lie over the coronal suture.

As described above, in some examples of the present technology the patient interface 3000 comprises a seal-forming structure 3100 in the form of a cradle cushion which lies generally under the nose and seals to an inferior periphery of the nose. The positioning and stabilising structure 3300 may be structured and arranged to pull the seal-forming structure 3100 into the patient's face under the nose with a sealing force vector that has a posterior and superior direction (e.g. a posterosuperior direction). A sealing force vector with a posterosuperior direction may facilitate the seal-forming structure 3100 forming a good seal to both the inferior periphery of the patient's nose and the anterior-facing surfaces of the patient's face on either side of the patient's nose and the upper lip.

For example, in many examples the positioning and stabilising structure 3300 may be configured such that the superior portions of the tubes 3350 lie across the patient's head slightly anterior to a superior-most point. For some patients this may result in the tubes 3350 being angled slightly anteriorly rather than aligned vertically (e.g. in the coronal plane) in order to lie within a slight recess at or proximate the coronal suture of the skull. In such an example, the tension in the strap 3310 could be adjusted by the patient to balance the forces and achieve the optimal sealing force vector.

In certain examples of the present technology, the tubes 3350 are configured to receive the strap 3310 at the locations superior to and proximate the patient's ears. If the strap 3310 connects to the tubes 3350 to high with respect to the patient's head, the strap 3310 may have a tendency to ride up the back of the patient's head. Additionally, the strap 3310 could form too large of an angle with respect to the superior portions of the headgear tubes 3350, resulting in the necessity for the patient to tighten the strap 3310 excessively, which could result in both excessive tension in the positioning and stabilising structure 3300 and make the strap 3310 more likely to ride up the back of the patient's head. Accordingly, it is advantageous for the connection between the strap 3310 and the tubes 3350 to be provided as low as possible but spaced from the top of the patient's ear sufficiently that upon tightening of the strap 3310, the tubes 3350 are not pulled into contact with the patient's ears.

### 5.3.3.1.2 Headgear Tube Fluid Connections

The two tubes 3350 are fluidly connected at their inferior ends to the plenum chamber 3200. In the examples of Figs. 43-47, the tubes 3350 form a fluid connection with the cradle cushion module 3150 and seal-forming structure 3100. In certain forms of the technology, the connection between the tubes 3350 and the cradle cushion module 3150 is achieved by connection of two rigid components so that the patient can easily connect the two components together in a reliable manner. The tactile feedback of a 're-assuring click' or like sound may be easy for a patient to use or for a patient to know that the tube has been correctly connected to the cradle cushion module 3150. In one form, the tubes 3350 are formed from silicone and the lower end of each of the silicone tubes 3350 is overmolded to a rigid connector made, for example, from polypropylene, polycarbonate, nylon or the like. The rigid connector may comprise a male mating feature configured to connect to a female mating feature on the cradle cushion module 3150. Alternatively, the rigid connector may comprise a female mating feature configured to connect to a male mating feature on the cradle cushion module 3150. The same manner of connection by which the tubes 3350 are connected to the cradle cushion module 3150 may also be applied to the connection between the tubes 3350 and the nasal cushion module 3150, or another plenum chamber 3200 or seal-forming structure 3100.

In another embodiment a compression seal is used to connect each tube 3350 to the cradle cushion module 3150. For example, a resiliently flexible (e.g. silicone) tube 3350 without the rigid connector may need to be squeezed slightly to reduce its diameter so that it can be jammed into a port in the plenum chamber 3200 and the inherent resilience of the silicone pushes the tube 3350 outwards to seal the tube 3350 in the port in an air-tight manner. In a hard-to-hard type engagement between the tube 3350 and port, a pressure activated seal such as a peripheral sealing flange may be used. When pressurised gas is supplied through the tubes 3350 the sealing flange is urged against the join between the tubes and the inner circumferential surface of the port of the plenum chamber 3200 to enhance the seal between them. If the port is soft and a rigid connector is provided to the tube 3350, the pressure activated seal as described earlier may also be used to ensure the connection is air-tight. In another example, each tube 3350 is formed from a resiliently flexible (e.g. silicone) material which is over moulded to a rigid connector such that the resiliently flexible material fits over the rigid connector and itself functions as a gasket to seal the connection between the tube 3350 and the cradle cushion module 3150 around a periphery of an air flow passage from the tube 3350 into the plenum chamber 3200 of the cradle cushion module 3150.

Similar connection mechanisms may be used to fluidly connect the tubes 3350 with a T-shaped top member defining the connection port 3600 or connectable to the connection port 3600 in some forms of the technology. In one embodiment, a swivel elbow connected at the connection port 3600 is rotatable in order to drive a port size adjustment mechanism that decreases or increases the size of the ports into which tubes 3350 are inserted in order to improve the fit of the tubes through an increase or decrease of compressive forces and to reduce unintended leakage.

### 5.3.4 Vent Assembly

In one form, the patient interface 3000 includes a vent assembly 3400 constructed and arranged to allow for the washout of exhaled gases, e.g. carbon dioxide.

In certain forms the vent assembly 3400 is configured to allow a continuous vent flow from an interior of the plenum chamber 3200 to ambient whilst the pressure within the plenum chamber is positive with respect to ambient. The vent assembly 3400 is configured such that the vent flow rate has a magnitude sufficient to reduce rebreathing of exhaled CO₂ by the patient while maintaining the therapeutic pressure in the plenum chamber in use.

The vent assembly 3400 may be located in the plenum chamber 3200, as shown in Figs. 43-57. The plenum chamber 3200 may include a receiving hole 3204 that receives the vent assembly 3400, which may include a vent body 3403. In the depicted examples, the plenum chamber 3200 includes a connection lip 3201 and the vent body 3403 includes a connection channel 3408 that receives the connection lip 3201 to connect the vent body 3403 to the plenum chamber 3200. The connection may be permanent, e.g., via an adhesive or overmoulding the plenum chamber 3200 onto the vent body 3403, or the vent body 3403 may be removably connected to the plenum chamber 3200 such that the connection lip 3201 and the connection channel 3408 are separable. Also, in another example, the vent body 3403 may have a connection lip that is received in a connection channel of the plenum chamber 3200. The plenum chamber 3200 may also include one or more orientation indication protrusions 3203 at the connection lip 3201, and the vent body 3403 may include one or more orientation indication recesses 3407 that receive corresponding ones of the orientation indication protrusions 3203 to indicate that the vent body 3403 has been assembled to the plenum chamber 3200 in the proper orientation. Also, in another example, the vent body 3403 may have one or more orientation indication protrusions that are received by one or more corresponding orientation indication recesses of the plenum chamber 3200 to indicate that the vent body 3403 has been assembled to the plenum chamber 3200 in the proper orientation. Additionally, the vent body 3403 and the receiving hole 3204 may have an oval shape.

The vent assembly 3400 may include vent holes 3402 that allow a continuous flow of gas to exit to atmosphere throughout the patient's respiratory cycle, as shown in Figs. 71-74. A vent cap 3401 may be connected to the vent body 3403, and the vent holes 3402 may be formed between the vent cap 3401 and the vent body 3403. The vent cap 3401 may be permanently joined to the vent body 3403 or the vent cap 3401 may removable. The vent body 3403 may also include vent body holes 3405 that allow gas to exit from within the plenum chamber 3200, and then through the vent holes 3402 to atmosphere.

A vent diffuser material 3404 may be positioned between the vent cap 3401 and the vent body 3403 to diffuse the flow of gas exiting through the vent body holes 3405 before it reaches atmosphere via the vent holes 3402 to reduce jetting and noise produced by the flow as it travels through the vent assembly 3400. The vent body 3403 may include a spacer 3406 to space the vent diffuser material 3404 away from the vent body holes 3405. The side of the vent diffuser material 3404 opposite the spacer 3406 may be supported against the vent cap 3401 to hold the vent diffuser material 3404 in position within the vent assembly 3400. The vent diffuser material 3404 may be a porous material, such as a foam or a network of fibers.

The vent body 3403 may be constructed from a material that is more rigid than the plenum chamber 3200. This may allow the vent body 3403 to resist collapse when subjected to a force so that the vent body holes 3405 and the vent holes 3402 are not occluded. The vent cap 3401 may also be constructed from a material that is more rigid than the plenum chamber 3200, which may be the same or different from the material of the vent body 3403.

The vent body 3403 may also include an attachment structure 3409 on a side that is positioned within the plenum chamber 3200 when the vent assembly 3400 is assembled to the plenum chamber 3200. The attachment structure 3409 may form a channel that receives an HMX rim 3806 of an HMX assembly 3800, which will be described below. The attachment structure 3409 may allow for the HMX rim 3806 to be connected to the vent assembly 3400 so that the vent assembly 3400 and the HMX assembly 3800 together form an HMX and vent assembly 3500. The HMX and vent assembly 3500, i.e., the vent assembly 3400 and the HMX assembly 3800 together, may be removed from the plenum chamber 3200 as a single unit. The attachment structure 3409 may also be constructed so that the HMX assembly 3800 can be removed from it. This may, for example, allow the vent assembly 3400 and/or the HMX assembly 3800 to be replaced at the same or different intervals.

### 5.3.5 Heat and Moisture Exchanger (HMX) Assembly

HMX assemblies 3800 according to examples of the present technology are shown in Figs. 48-104. As described above, the HMX assembly 3800 is connected to the vent assembly 3400 to form the HMX and vent assembly 3500, which may then be attached to the plenum chamber 3200.

Figs. 58-62, 77-80, and 88-93 show examples of the HMX and vent assembly 3500. In these examples, the vent body 3403 includes the attachment structure 3409, which may be in the form of multiple structures on a posterior side of the vent body 3403, that receives the HMX rim 3806. Since the HMX frame 3802 and the HMX rim 3806, as well as the HMX strut 3804 where applicable, may be constructed of a single piece of homogeneous material that may be relatively flexible, like silicone rubber or thermoplastic elastomer (TPE) and the vent body 3403, including the attachment structure 3409, may be constructed of a relatively rigid material, the HMX rim 3806 may be flexed and stretched to fit over the attachment structure 3409 to attach the HMX frame 3802 to the vent body 3403. The HMX rim 3806 may also form an HMX rim hole 3807 that gases may pass through before reaching the vent body holes 3405. The HMX rim 3806 may have a shape that similar to that of the vent body 3403, which in the first two of the present examples is an oval and polygon in the third. The HMX material 3850 may have a shape that corresponds to the HMX rim 3806 such that the HMX material 3850 fits completely within the HMX rim 3806.

Extending from the HMX rim 3806 there may be one or more HMX struts 3804 that connect the HMX rim 3806 to the HMX frame 3802. Alternatively, the HMX struts 3804 may be omitted and the HMX frame 3802 is directly connected to the HMX rim 3806. The first example, in Figs. 48-70, includes HMX struts 3804 that are straight. The second example, in Figs. 75-82, includes features similar to the first example, but the HMX struts 3804 are bent. The third example, in Figs. 83-104, does not include HMX struts 3804. In the first and second examples, there are two HMX struts 3804 and they allow the HMX frame 3802, which supports an HMX material 3850, to pivot laterally in response to forces applied by the patient's face, for example, during movement. The straight HMX struts 3804 of the first example may be flexible due to the nature of the relatively flexible material, and the bent HMX struts 3804 of the second example may allow further flexibility because the bend permits directed buckling. The bent HMX struts 3804 may also provide a springing function that absorbs forces against the HMX frame 3802 to decouple the HMX frame 3802 from the HMX rim 3806 and the vent body 3403, which might otherwise disrupt sealing because the seal-forming structure 3100 may be connected to the plenum chamber 3200 which may in turn be connected to the vent body 3403. The attachment structure 3409 may also be formed with a number of slots 3410 corresponding to the number of HMX struts 3804 to receive the HMX struts 3804 when the HMX rim 3806 is assembled to the vent body 3403.

The flexibility of the HMX frame 3802 and the HMX material 3850 may also permit relatively easy assembly to the plenum chamber 3200 because these parts can be deformed to fit through the receiving hole 3204 and then return to their original size and shape within the plenum chamber 3200. This may allow the HMX assembly 3800 to be used with patient interfaces 3000 that have a relatively small outer profile and cannot accommodate a large receiving hole 3204, such as nasal cradles and nasal pillows, due to their inherently small size. As can be seen in the cross-sectional views of Figs. 53, 54, 85-87, and 102-104, the HMX assembly 3800 is larger than the receiving hole 3204, it can still be inserted within the plenum chamber 3200 because it is flexible. Also, if the plenum chamber 3200 is constructed from a relatively flexible material, such as silicone rubber, the receiving hole 3204 can also be deformed to allow the HMX assembly 3800 to be inserted therethrough. These same aspects similarly facilitate disassembly of the HMX assembly 3800 from the plenum chamber 3200.

The HMX material 3850 positioned on or supported by the HMX frame 3802 may be foam, e.g., open-cell foam, or paper. The foam or paper may be further treated with a salt to adsorb more moisture than the porosity of foam or paper alone is capable of.

The HMX material 3850 may be relatively flexible. The flexible HMX material 3850, along with the flexible HMX frame 3802, may allow the HMX assembly 3800 to conform to a number of different shapes and/or sizes of seal-forming structure 3100. When the HMX assembly 3800 is positioned inside of the plenum chamber 3200, the HMX frame 3802 and/or the HMX material 3850 may be in contact with the seal-forming structure 3100 in an undeformed state or the HMX frame 3802 and the HMX material 3850 may be positioned close to the seal-forming structure 3100. In either scenario, when the patient dons the patient interface 3000, the seal-forming structure 3100 may be deformed into the HMX frame 3802 and/or the HMX material 3850 by the patient's face contacting a patient-contacting surface 3101, and the flexible nature of the HMX frame 3802 and the HMX material 3850 allow one or both of these components to deform to minimize discomfort for the patient. The HMX assembly 3800 may be employed in a one-size-fits-all approach in that the HMX and vent assembly 3500 of one size can be assembled to different patient interface 3000 configurations (e.g., nasal cradle, nasal pillows, nasal, oronasal) that may further have different sizes (e.g., small, medium, large), while the flexible construction allows the HMX and vent assembly 3500 to be comfortable for the patient in all applicable patient interface 3000 arrangements. The HMX frame 3802 and the HMX material 3850 may also both be shaped with a positive curvature on their respective posterior sides to more easily and more comfortably conform to the patient's face. The HMX material 3850 may have an anterior surface 3854 that has a negative curvature. The HMX material 3850 may have a posterior surface 3852 that has a positive curvature. The HMX material 3850 may have a thickness that is uniform between the posterior surface 3852 and the anterior surface 3854, or the thickness may vary, e.g., thinnest in the middle and thicker at the lateral sides or thickest in the middle and thinner at the lateral sides. Also, the posterior surface 3852 may be directly accessible through one or more seal - forming structure holes 3102 so that humidified and pressurized air passing through the HMX material 3850 exits through the seal - forming structure holes 3102 for inhalation by the patient and also to allow exhaled gas to reach the posterior surface 3852 by passing back through the seal - forming structure holes 3102.

The HMX material 3850 may be attached to the HMX frame 3802 around a peripheral surface 3856 of the HMX material 3850. In the depicted examples, the peripheral surface 3856 does not extend beyond the HMX frame 3802, but in alternative examples the peripheral surface 3856 may extend beyond the HMX frame 3802. The HMX material 3850 may be attached to the HMX frame 3802 by one or more of the following: an adhesive, overmoulding the HMX frame 3802 onto the HMX material 3850, and retainers to hold the HMX material 3850 against the HMX frame 3802. For example, where the HMX frame 3802 is constructed from TPE, the HMX frame 3802 may be overmoulded onto the HMX material 3850, and where the HMX frame 3802 is constructed from silicone rubber, the HMX frame 3802 may be glued to the HMX material 3850. The connection between the HMX material 3850 and the HMX frame 3802 may be permanent or it may be removable. When the HMX material 3850 has passed its useful life (after a predetermined amount of use, its ability to adsorb and desorb moisture may begin to deteriorate), the HMX material 3850 and the HMX frame 3802 may be disposed of together and replaced with new such parts as well.

The seal-forming structure 3100 and/or the plenum chamber 3200 may also have internal features that hold the HMX frame 3802 and the HMX material 3850 in a predetermined position to optimize performance of the HMX material 3850 and to optimize anthropometric fit during therapy. Figs. 85-87 and 102-104 show a sealing lip 3103 engaged with the HMX frame 3802 to seal around its entire periphery. This arrangement may force all of the incoming flow of pressurized air from the tube connectors 3202 to either discharge directly to atmosphere through the vent assembly 3400 or to pass through the HMX material 3850 to reach the patient, after passing through the HMX frame hole 3803. This may ensure that all of the pressurized air that reaches the patient can adsorb moisture from the HMX material 3850 before reaching the patient's airways. Also, all gas exhaled by the patient must pass through the HMX material 3850 in this arrangement before passing through the vent assembly 3400 to atmosphere. In a further example, one or more sealing lip bypass openings 3104 may be formed in the sealing lip 3103 so that the sealing lip 3103 does not engage and seal around the entire periphery of the HMX frame 3802. This allows some of the pressurized air traveling to the patient and some of the gas exhaled by the patient to bypass the HMX material 3850. The presence or absence of one or more sealing lip bypass openings 3104, as well as size and shape of sealing lip bypass openings 3104 if present, allow for optimization of the humidification of the incoming flow of pressurized air.

### 5.3.6 Decoupling structure(s)

In one form the patient interface 3000 includes at least one decoupling structure, for example, a swivel or a ball and socket. For example, the patient interface 3000 shown in Figs. 43-47comprises an elbow 3610 configured the swivel with respect to the positioning and stabilising structure 3300. In this example the elbow 3610 is configured to swivel about an axis concentric with a circular opening in the positioning and stabilising structure 3300. In some examples of the present technology, the elbow 3610 may form part of a ball and socket joint to the positioning and stabilising structure 3300. For example, a ring having a partially spherical inner surface may be provided to the positioning and stabilising structure 3300 and may be configured to receive the elbow 3610. The elbow 3610 may have partially spherical outer surface complimentary to the partially spherical inner surface of the ring, thereby enabling the elbow 3610 to swivel with respect to the ring in a plurality of axes.

### 5.3.7 Connection port

Connection port 3600 allows for connection to the air circuit 4170. In the exemplary patient interface 3000 shown in Figs. 43-47, the elbow 3610 forms part of the connection port 3600. The elbow 3610, as a decoupling structure, decouples movement of the air circuit 4170 from the positioning and stabilising structure 3300 in order to reduce tube drag on the positioning and stabilising structure 3300.

### 5.3.8 Forehead support

In one form, the patient interface 3000 includes a forehead support 3700. In another form, the patient interface 3000 does not include a forehead support 3700. Advantageously, the exemplary patient interface 3000 shown in Figs. 43-47comprises a positioning and stabilising structure 3300 that is able to hold the seal-forming structure 3100 in sealing position without connection to a forehead support or any frame or strap members that lie in front of the patient's face at eye level.

### 5.3.9 Anti-asphyxia valve

In one form, the patient interface 3000 includes an anti-asphyxia valve.

### 5.3.10 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to pressurize a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

The RPT device may have an external housing 4010. The external housing 4010 may be formed in two parts, an upper portion 4012 and a lower portion 4014. Furthermore, the external housing 4010 may include one or more panel(s) 4015. The RPT device 4000 may comprise a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124, and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016. The pneumatic block 4020 may include one or more pneumatic components 4100.

The RPT device 4000 may have an electrical power supply 4210, one or more input devices 4220, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH₂O to about 20 cmH₂O, or in other forms up to about 30 cmH₂O when delivering respiratory pressure therapy. The blower may be as described in any one of the following patents or patent applications the contents of which are incorporated herein by reference in their entirety: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

### 5.5.1 Supplementary gas delivery

In one form of the present technology, supplementary gas 4180, e.g. oxygen, is delivered to one or more points in the pneumatic path, such as upstream of the pneumatic block 4020, to the air circuit 4170, and/or to the patient interface 3000 or 3800.

### 5.6 HUMIDIFIER

### 5.6.1 Humidifier overview

In one form of the present technology there is provided a humidifier 5000 to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir, a humidifier inlet to receive a flow of air, and a humidifier outlet to deliver a humidified flow of *air.* In some forms, an inlet and an outlet of the humidifier reservoir may be the humidifier inlet and the humidifier outlet respectively. The humidifier 5000 may further comprise a humidifier base, which may be adapted to receive the humidifier reservoir and comprise a heating element.

### 5.7 BREATHING WAVEFORMS

Fig. 42 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.8 GLOSSARY

For the purposes of the present technology disclosure, in certain forms of the present technology, one or more of the following definitions may apply. In other forms of the present technology, alternative definitions may apply.

### 5.8.1 General

*Air*: In certain forms of the present technology, air may be taken to mean atmospheric air, and in other forms of the present technology air may be taken to mean some other combination of breathable gases, e.g., atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g., the humidity in the room where a patient is sleeping. Such ambient humidity may be different to the humidity outside the room where a patient is sleeping.

In another example, ambient pressure may be the pressure immediately surrounding or external to the body.

In certain forms, ambient noise (e.g., acoustic) may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g., from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.8.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC^{®} (included in the range of products sold under this trademark), manufactured by DuPont. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.8.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.8.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.8.3 Anatomy

### 5.8.3.1 Anatomy of the face

*Ala:* the external outer wall or "wing" of each nostril (plural: alar)

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 5.8.3.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.8.3.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.8.4 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g., about 360 degrees. In certain forms an elbow may be removable from a mating component, e.g., via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.8.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g., outer) surface, and a separate non-face-contacting (e.g., underside or inner) surface. In another example, a structure may comprise a first surface and a second surface.

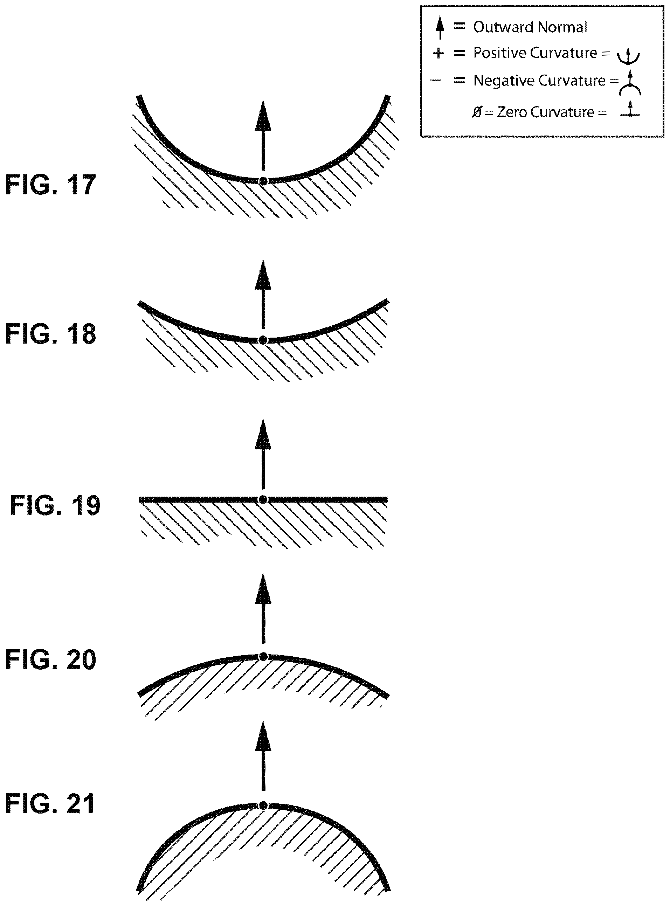

To facilitate describing the shape of the three-dimensional structures and the surfaces, we first consider a cross-section through a surface of the structure at a point, *p.* See Fig. 17 to Fig. 21, which illustrate examples of cross-sections at point *p* on a surface, and the resulting plane curves. Figs. 17 to 21 also illustrate an outward normal vector at *p.* The outward normal vector at *p* points away from the surface. In some examples we describe the surface from the point of view of an imaginary small person standing upright on the surface.

### 5.8.5.1 Curvature in one dimension

The curvature of a plane curve at *p* may be described as having a sign (e.g., positive, negative) and a magnitude (e.g., 1/radius of a circle that just touches the curve at *p*)*.*

*Positive curvature:* If the curve at *p* turns towards the outward normal, the curvature at that point will be taken to be positive (if the imaginary small person leaves the point *p* they must walk uphill). See Fig. 17 (relatively large positive curvature compared to Fig. 18) and Fig. 18 (relatively small positive curvature compared to Fig. 17). Such curves are often referred to as concave.

*Zero curvature:* If the curve at *p* is a straight line, the curvature will be taken to be zero (if the imaginary small person leaves the point *p*, they can walk on a level, neither up nor down). See Fig. 19.

*Negative curvature:* If the curve at *p* turns away from the outward normal, the curvature in that direction at that point will be taken to be negative (if the imaginary small person leaves the point *p* they must walk downhill). See Fig. 20 (relatively small negative curvature compared to Fig. 21) and Fig. 21 (relatively large negative curvature compared to Fig. 20). Such curves are often referred to as convex.

### 5.8.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g., relatively small. The plane curves in Figs. 17 to 21 could be examples of such multiple cross-sections at a particular point.

*Principal curvatures and directions:* The directions of the normal planes where the curvature of the curve takes its maximum and minimum values are called the principal directions. In the examples of Fig. 17 to Fig. 21, the maximum curvature occurs in Fig. 17, and the minimum occurs in Fig. 21, hence Fig. 17 and Fig. 21 are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g., both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.8.5.3 Space curves

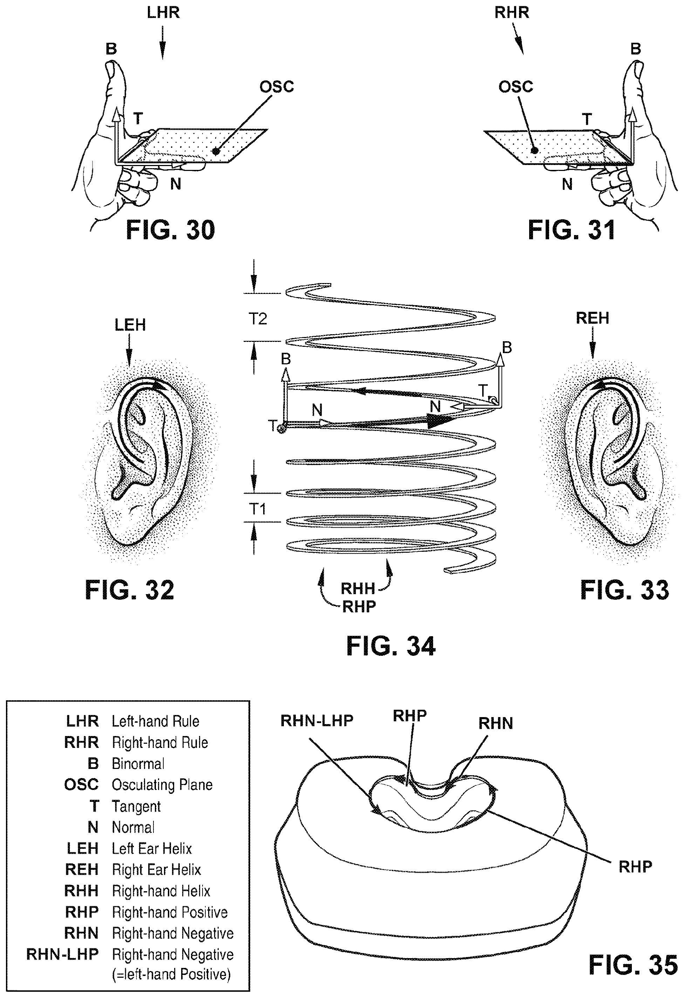

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 32. A typical human right ear comprises a helix, which is a right-hand helix, see Fig. 33. Fig. 34 shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g., Fig. 31), or alternatively by a left-hand rule (Fig. 30).

*Osculating plane:* The plane containing the unit tangent vector and the unit principal normal vector. See Figures 30 and 31.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g., a gently sloping helical path). A space curve which deviates a relatively large amount from the osculating plane will have a relatively large magnitude of torsion (e.g., a steeply sloping helical path). With reference to Fig. 34, since T2>T1, the magnitude of the torsion near the top coils of the helix of Fig. 34 is greater than the magnitude of the torsion of the bottom coils of the helix of Fig. 34

With reference to the right-hand rule of Fig. 31, a space curve turning towards the direction of the right-hand binormal may be considered as having a right-hand positive torsion (e.g., a right-hand helix as shown in Fig. 34). A space curve turning away from the direction of the right-hand binormal may be considered as having a right-hand negative torsion (e.g., a left-hand helix).

Equivalently, and with reference to a left-hand rule (see Fig. 30), a space curve turning towards the direction of the left-hand binormal may be considered as having a left-hand positive torsion (e.g., a left-hand helix). Hence left-hand positive is equivalent to right-hand negative. See Fig. 35.

### 5.8.5.4 Holes

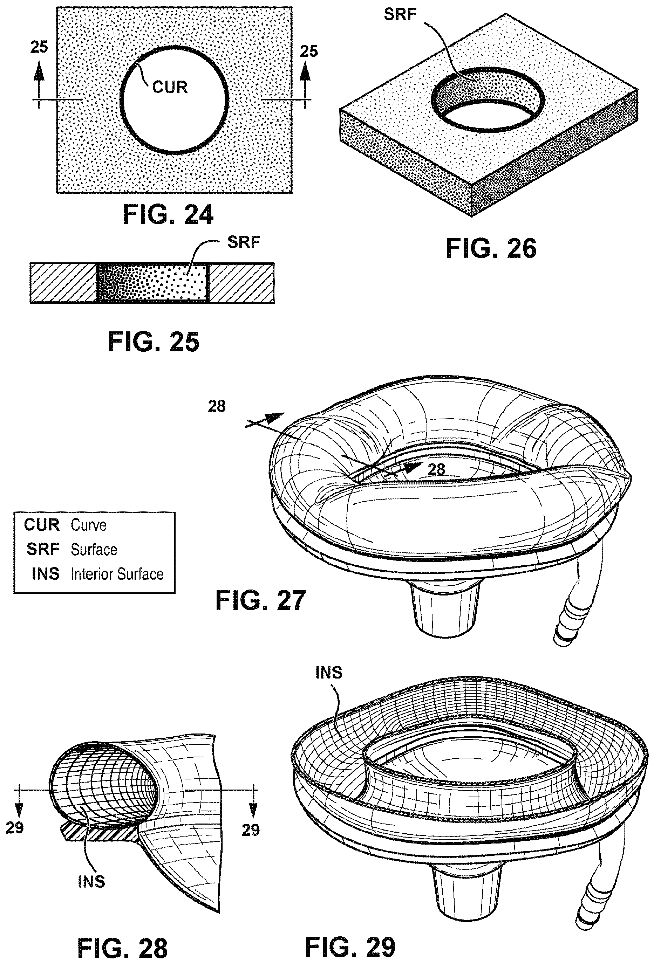

A surface may have a one-dimensional hole, e.g. a hole bounded by a plane curve or by a space curve. Thin structures (e.g., a membrane) with a hole, may be described as having a one-dimensional hole. See for example the one dimensional hole in the surface of structure shown in Fig. 24, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 27 and the example cross-sections therethrough in Fig. 28 and Fig. 29, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 26, bounded by a surface as shown.

### 5.9 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

### 5.10 REFERENCE SIGNS LIST

| | |
|---|---|
| patient | 1000 |
| bed partner | 1100 |
| patient interface | 3000 |
| seal - forming structure | 3100 |
| seal-forming structure hole | 3102 |
| sealing lip | 3103 |
| sealing lip bypass opening | 3104 |
| cradle cushion module | 3150 |
| pillows cushion module | 3160 |
| nasal pillows | 3165 |
| plenum chamber | 3200 |
| connection lip | 3201 |
| tube connector | 3202 |
| orientation indication protrusion | 3203 |
| receiving hole | 3204 |
| chord | 3210 |
| superior point | 3220 |
| inferior point | 3230 |
| positioning and stabilising structure | 3300 |
| strap | 3310 |
| tube | 3350 |
| non - extendable tube section | 3363 |
| sleeve | 3364 |
| fluid connection opening | 3390 |
| vent assembly | 3400 |
| vent cap | 3401 |
| vent hole | 3402 |
| vent body | 3403 |
| vent diffuser material | 3404 |
| vent body hole | 3405 |
| spacer | 3406 |
| orientation indication recess | 3407 |
| connection channel | 3408 |
| attachment structure | 3409 |
| slot | 3410 |
| HMX and vent assembly | 3500 |
| connection port | 3600 |
| elbow | 3610 |
| forehead support | 3700 |
| HMX assembly | 3800 |
| HMX frame | 3802 |
| HMX frame hole | 3803 |
| HMX strut | 3804 |
| HMX rim | 3806 |
| HMX rim hole | 3807 |
| HMX material | 3850 |
| posterior surface | 3852 |
| anterior surface | 3854 |
| peripheral surface | 3856 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| pneumatic components | 4100 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| supplementary gas | 4180 |
| electrical components | 4200 |
| PCBA | 4202 |
| electrical power supply | 4210 |
| input devices | 4220 |
| central controller | 4230 |
| transducer | 4270 |
| humidifier | 5000 |

The following aspects are preferred embodiments of the invention.
1. A patient interface comprising:
   a plenum chamber pressurisable to a therapeutic pressure of at least 4 cmH2O above ambient air pressure by a flow of air at the therapeutic pressure for breathing by a patient, the plenum chamber further comprising two plenum chamber connectors, each of the plenum chamber connectors being positioned on a corresponding lateral side of the plenum chamber and configured to receive the flow of air at the therapeutic pressure;
   a seal-forming structure connected to the plenum chamber, the seal-forming structure being constructed and arranged to seal with a region of the patient's face that at least partly surrounds an entrance to the patient's airways, the seal-forming structure having a hole therein to deliver the flow of air at the therapeutic pressure to at least the patient's nares during use, the seal-forming structure being constructed and arranged to maintain the therapeutic pressure within the plenum chamber throughout the patient's respiratory cycle in use;
   a positioning and stabilising structure configured to hold the seal-forming structure in a therapeutically effective position on the patient's head, the positioning and stabilising structure further comprising two conduits, each of the conduits being configured to be positioned on a corresponding lateral side of the patient's head in use and being configured to connect to a corresponding one of the plenum chamber connectors;
   a vent assembly connected to the plenum chamber and having a plurality of vent holes configured to allow a continuous flow of gases exhaled by the patient from within the plenum chamber to ambient throughout the patient's respiratory cycle, the vent holes being sized and shaped to maintain the therapeutic pressure in the plenum chamber in use; and
   a heat and moisture exchanger (HMX) assembly connected to the vent assembly, the HMX assembly including an HMX frame and a material positioned on the HMX frame, the material being configured to adsorb water vapour from gases exhaled by the patient and desorb water vapour into the flow of air at the therapeutic pressure, the material being supported by the HMX frame within the plenum chamber such that at least a portion of the flow of air at the therapeutic pressure entering the plenum chamber from the plenum chamber connectors passes through the material before entering the patient's airways;
   wherein the patient interface is configured to leave the patient's mouth uncovered, or the seal-forming structure is configured to seal around the patient's nose and mouth and the patient interface is configured to allow the patient to breath from ambient in the absence of the flow of air at the therapeutic pressure.
2. The patient interface of aspect 1, wherein the material further comprises foam or paper.
3. The patient interface of one of aspect 1 or 2, wherein the material further comprises a salt applied to a surface of the material.
4. The patient interface of one of aspects 1 to 3, wherein the material is removable from the HMX frame.
5. The patient interface of one of aspects 1 to 4, wherein the HMX assembly is removable from within the plenum chamber.
6. The patient interface of one of aspects 1 to 5, wherein the vent assembly is removably connected to the plenum chamber.
7. The patient interface of one of aspects 1 to 6, wherein the vent assembly and the HMX assembly are removable together from the plenum chamber.
8. The patient interface of one of aspects 1 to 7, wherein the HMX assembly is removable from the vent assembly.
9. The patient interface of one of aspects 1 to 8, wherein the vent assembly comprises a vent body and a vent cap attached to the vent body.
10. The patient interface of one of aspects 1 to 9, wherein the vent holes are formed between the vent body and the vent cap.
11. The patient interface of one of aspects 1 to 10, wherein the vent body further comprises vent body holes configured to allow the continuous flow of gases exhaled by the patient to pass through the vent body and then to atmosphere through the vent holes.
12. The patient interface of one of aspects 1 to 11, wherein the vent assembly includes a vent diffuser material positioned between the vent body and the vent cap.
13. The patient interface of one of aspects 1 to 12, wherein the vent body includes a spacer to space the vent diffuser material away from the vent body holes and against the vent cap.
14. The patient interface of one of aspects 1 to 13, wherein one of the plenum chamber and the vent body includes one or more orientation indication protrusions,
   wherein the other of the plenum chamber and the vent body includes one or more orientation indication recesses configured to receive corresponding ones of the orientation indication protrusions when the vent body is attached to the plenum chamber.
15. The patient interface of one of aspects 1 to 14, wherein one of the plenum chamber and the vent body includes a connection lip,
   wherein the other of the plenum chamber and the vent body includes a connection channel configured to receive the connection lip to removably attach the vent body to the plenum chamber.
16. The patient interface of one of aspects 1 to 15, wherein the plenum chamber includes a receiving hole configured to receive the vent assembly and through which the HMX assembly is inserted inside of the plenum chamber.
17. The patient interface of one of aspects 1 to 16, wherein the HMX frame is constructed from a flexible material.
18. The patient interface of one of aspects 1 to 17, wherein the flexible material is silicone rubber or thermoplastic elastomer.
19. The patient interface of one of aspects; 1 to 18, wherein the material is positioned within the plenum chamber such that the flow of air at the therapeutic pressure must pass through the material before entering the patient's airways.
20. The patient interface of one of aspects 1 to 19, wherein the material is positioned within the plenum chamber such that gas exhaled by the patient must pass through the material before exiting to ambient through the vent holes.
21. The patient interface of one of aspects 1 to 20, wherein the material is positioned within the plenum chamber such that at least a portion of the flow of air at the therapeutic pressure bypasses the material before entering the patient's airways.
22. The patient interface of one of aspects 1 to 21, wherein the material is positioned within the plenum chamber such that at least a portion of the gas exhaled by the patient bypasses the material before exiting to ambient through the vent holes.
23. The patient interface of one of aspects; 1 to 22, wherein the HMX frame forms an HMX frame hole, and
   wherein a peripheral portion of the material is positioned on the HMX frame such that gases flowing through the material pass through the HMX frame hole.
24. The patient interface of one of aspects 1 to 23, wherein the HMX frame includes an HMX rim connected to the vent assembly.
25. The patient interface of one of aspects 1 to 24, wherein the HMX frame includes one or more HMX struts joined to the HMX rim.
26. The patient interface of one of aspects 1 to 25, wherein each of the HMX struts is straight.
27. The patient interface of one of aspects 1 to 26, wherein each of the HMX struts is bent.
28. The patient interface of one of aspects 1 to 27, wherein the HMX rim is joined directly to the HMX frame.
29. The patient interface of one of aspects 1 to 28, wherein the vent body includes an attachment structure configured to receive the HMX rim to attach the HMX frame to the vent body.
30. The patient interface of one of aspects 1 to 29, wherein one or more slots are formed through the attachment structure, each of the slots being configured to receive a corresponding one of the HMX struts.
31. The patient interface of one of aspects 1 to 30, wherein the material is permanently attached to the HMX frame.
32. The patient interface of one of aspects; 1 to 31, wherein the material is attached to the HMX frame by one or more of the following: an adhesive, overmoulding the HMX frame onto the material, and retainers to hold the material against the HMX frame.
33. The patient interface of one of aspects 1 to 32, wherein the material further comprises a posterior surface that faces the patient's face in use, the posterior surface being positively curved to avoid contact with the patient's face during use.
34. The patient interface of one of aspects; 1 to 33, wherein the seal-forming structure comprises a sealing lip configured to seal against the HMX frame.
35. The patient interface of one of aspects 1 to 34, wherein the sealing lip includes a sealing lip bypass opening configured to allow at least a portion of the flow of air at the therapeutic pressure to bypass the material before entering the patient's airways and to allow at least a portion of the gas exhaled by the patient to bypass the material before exiting to ambient through the vent holes.
36. The patient interface of one of aspects 1 to 35, wherein the sealing lip is configured to seal around the entire perimeter of the HMX frame such that all of the flow of air at the therapeutic pressure passes through the material before entering the patient's airways and all of the gas exhaled by the patient passes through the material before exiting to ambient through the vent holes.
37. The patient interface of one of aspects 1 to 36, wherein the seal-forming structure is configured to leave the patient's mouth uncovered and the seal-forming structure further comprises nasal pillows or a nasal cradle.
38. The patient interface of one of aspects 1 to 37, wherein the seal-forming structure is configured to seal around the patient's nose and mouth and the seal-forming structure further comprises a nasal hole or two nasal holes that are configured to pneumatically communicate with the patient's nares in use and a mouth hole that is configured to pneumatically communicate with the patient's mouth in use.
39. A respiratory pressure therapy (RPT) system comprising:
   the patient interface of any one of claims 1 to 38;
   a respiratory pressure therapy (RPT) device configured to generate the flow of air at the therapeutic pressure; and
   an air circuit configured to connect the RPT device and the patient interface to direct the flow of air at the therapeutic pressure from the RPT device to the patient interface,
   wherein the RPT system does not include a humidifier.

## Claims

1. A vent and heat and moisture exchanger (HMX) assembly (3500) comprising:
a vent assembly (3400) configured to be connected to a plenum chamber (3200) of a patient interface (3000) and having a plurality of vent holes (3402) configured to allow a continuous flow of gases exhaled by a patient from within the plenum chamber (3200) to ambient throughout the patient's respiratory cycle, the vent holes (3402) being sized and shaped to maintain a therapeutic pressure in the plenum chamber (3200) in use; and
an HMX assembly (3800) connected to the vent assembly (3400) such that the HMX assembly (3800) and the vent assembly (3400) are removable from the plenum chamber (3200) together as a single unit, the HMX assembly (3800) including an HMX frame (3802) and a material (3850) positioned on the HMX frame (3802), the material (3850) being configured to adsorb water vapour from gases exhaled by the patient and desorb water vapour into a flow of air at the therapeutic pressure, the material (3850) being supported by the HMX frame (3802) such that when the vent assembly (3400) is connected to the plenum chamber (3200), the material (3850) is positioned within the plenum chamber (3200) to allow at least a portion of the flow of air at the therapeutic pressure to pass through the material (3850) before entering the patient's airways,
wherein the HMX frame (3802) is constructed from a flexible material.

2. The vent and HMX assembly (3500) of claim 1, wherein the material (3850) further comprises foam or paper.

3. The vent and HMX assembly (3500) of claim 1 or 2, wherein the material (3850) is removable from the HMX frame (3802) or permanently attached to the HMX frame (3802).

4. The vent and HMX assembly (3500) of one of claims 1 to 3, wherein the vent assembly (3400) is configured to be removably connected to the plenum chamber (3200).

5. The vent and HMX assembly (3500) of one of claims 1 to 4, wherein the HMX assembly (3800) is removable from the vent assembly (3400).

6. The vent and HMX assembly (3500) of one of claims 1 to 5, wherein the vent assembly (3400) comprises a vent body (3403) and a vent cap (3401) attached to the vent body (3403).

7. The vent and HMX assembly (3500) of claim 6, wherein the HMX frame (3802) includes an HMX rim (3806) connected to the vent assembly (3400), and
wherein the vent body (3403) includes an attachment structure (3409) configured to receive the HMX rim (3806) to attach the HMX frame (3802) to the vent body (3403).

8. The vent and HMX assembly (3500) of claim 7, wherein the HMX frame (3802) includes one or more HMX struts (3804) joined to the HMX rim (3806), and
wherein one or more slots (3410) are formed through the attachment structure (3409), each of the slots (3410) being configured to receive a corresponding one of the HMX struts (3804).

9. The vent and HMX assembly (3500) of one of claims 1 to 6, wherein the HMX frame (3802) includes an HMX rim (3806) connected to the vent assembly (3400).

10. The vent and HMX assembly (3500) of one of claims 1 to 9, wherein the HMX frame (3802) forms an HMX frame hole (3803), and
wherein a peripheral portion of the material (3850) is positioned on the HMX frame (3802) such that gases flowing through the material (3850) pass through the HMX frame hole (3803).

11. The vent and HMX assembly (3500) of claim 10, wherein the HMX frame (3802) includes one or more HMX struts (3804) joined to the HMX rim (3806).

12. The vent and HMX assembly (3500) of one of claims 1 to 11, wherein the material (3850) further comprises a posterior surface (3852) that faces the patient's face in use, the posterior surface (3852) being positively curved to avoid contact with the patient's face during use.

13. A patient interface (3000) comprising:
a plenum chamber (3200) pressurisable to a therapeutic pressure of at least 4 cmH2O above ambient air pressure by a flow of air at the therapeutic pressure for breathing by a patient, the plenum chamber (3200) further comprising a plenum chamber opening configured to receive the flow of air at the therapeutic pressure;
a seal-forming structure (3100) connected to the plenum chamber (3200), the seal-forming structure (3100) being constructed and arranged to seal with a region of the patient's face that at least partly surrounds an entrance to the patient's airways, the seal-forming structure (3100) having a hole (3102) therein to deliver the flow of air at the therapeutic pressure to at least the patient's nares during use, the seal-forming structure (3100) being constructed and arranged to maintain the therapeutic pressure within the plenum chamber (3200) throughout the patient's respiratory cycle in use;
a positioning and stabilising structure comprising a tie configured to hold the seal-forming structure (3100) in a therapeutically effective position on the patient's head; and
the vent and HMX assembly (3500) of one of claims 1 to 12,
wherein the patient interface (3000) is configured to leave the patient's mouth uncovered, or the seal-forming structure (3100) is configured to seal around the patient's nose and mouth and the patient interface (3000) is configured to allow the patient to breath from ambient in the absence of the flow of air at the therapeutic pressure.

14. The patient interface (3000) of claim 13, wherein the seal-forming structure (3100) comprises a sealing lip (3103) configured to seal against the HMX frame (3802).

15. The patient interface (3000) of claim 13 or 14, wherein the plenum chamber (3200) includes a receiving hole (3204) configured to receive the vent assembly (3400) and through which the HMX assembly (3800) is inserted inside of the plenum chamber (3200).
